# EUROPÄISCHE PATENTSCHRIFT

(11) **EP 4 606 301 B1**
(45) Veröffentlichungstag und Bekanntmachung des Hinweises auf die Patenterteilung: **21.10.2026**
(21) Anmeldenummer: 24159090.0
(22) Anmeldetag: 22.02.2024
(51) Int. Cl.: A61B 5/055, A61B 6/04

(54) **PATIENTENSITZ UND MAGNETRESONANZGERÄT**
PATIENT SEAT AND MAGNETIC RESONANCE APPARATUS
SIÈGE DE PATIENT ET APPAREIL DE RÉSONANCE MAGNÉTIQUE

(43) Veröffentlichungstag der Anmeldung: 27.08.2025
(73) Patentinhaber: Siemens Healthineers AG, 91301 Forchheim (DE)
(72) Erfinder: Greiser, Andreas, 91054 Erlangen (DE); Hausotte, Annemarie, 91052 Erlangen (DE); Keil, Miriam, 91056 Erlangen (DE); Krüger, Gunnar, 4102 Binningen (CH); Lauer, Lars, 91077 Neunkirchen (DE)
(74) Vertreter: Siemens Healthineers Patent Attorneys

(56) Entgegenhaltungen:
- CN-A- 116 725 514
- DE-A1- 102018 212 091
- DE-A1- 102021 205 916
- US-A- 4 961 208
- US-A1- 2018 011 153
- US-A1- 2021 156 936
- US-A1- 2022 308 140

## Beschreibung

Unabhängig vom grammatikalischen Geschlecht eines bestimmten Begriffes sind Personen mit männlicher, weiblicher oder anderer Geschlechteridentität mit umfasst.

Für den Einsatz von dedizierten Magnetresonanzgeräten, insbesondere dedizierten Kopfscannern oder dedizierten Dentalscannern, in kleineren medizinischen Einrichtungen und Praxen sind ein möglichst geringer Platzbedarf, eine möglichst einfache und zeiteffiziente Patientenpositionierung, sowie ein hoher Patientenkomfort von entscheidender Bedeutung. Üblicherweise geht eine Reduktion der Größe von Magnetresonanzgeräten mit der Verkleinerung eines Bildgebungsvolumens einher, wodurch die Anforderungen an die Genauigkeit der Patientenpositionierung steigen. Darüber hinaus erfordert eine genaue Patientenpositionierung geschultes Personal, was insbesondere für kleinere medizinische Einrichtungen, sowohl aus logistischer als auch aus finanzieller Sicht, ein Problem darstellen kann.

Magnetresonanzgeräte verwenden heute mit sehr wenigen Ausnahmen horizontal ausgerichtete Patiententische mit einer im Wesentlichen planaren Liegefläche. Bei solchen konventionellen Patiententischen wird eine Empfangsspule in einem zweistufigen Arbeitsablauf zunächst manuell an einer diagnostisch relevanten Körperregion eines Patienten angeordnet, bevor ein Mittenversatz der Empfangsspule von einem gegebenen Referenzpunkt für eine nachfolgende Isozentrierung durch Bewegung des Patiententisches entlang einer Zugangsrichtung des Magnetresonanzgeräts (typischerweise einer Längsachse eines Patientenaufnahmebereichs oder einer Z-Richtung des Magnetresonanzgeräts) bestimmt wird. Konventionelle Patiententische stellen sich gerade bei kleineren medizinischen Einrichtungen und Praxen als ungeeignet heraus, da diese relativ viel Raum benötigen und mit komplexen Arbeitsabläufen verbunden sind. Weiterhin ist eine "präzise" Isozentrierung bislang nur parallel zur Längsachse des Patiententisches, aber nicht entlang weiterer Raumrichtungen, möglich. Bekannte Patientensitze, wie sie teilweise bei Extremitäten-Scannern verwendet werden, ermöglichen zwar eine Reduktion des Platzbedarfs, reduzieren aber nicht den mit der Patientenpositionierung verbundenen Arbeitsaufwand.

Die Druckschrift CN 116 725 514 A betrifft einen für die Magnetresonanztomographie geeigneten Bettstuhl sowie eine entsprechende Vorrichtung, die eine Sitzplatte, eine Rückenplatte, eine Beinstützplatte und eine anpassbare Hochfrequenzspulenvorrichtung umfasst.

In der Druckschrift US 4 961 208 A wird eine Vorrichtung beschrieben, welche einen auf einem Hauptrahmen gelagerten Gantry und einen Personenhalter umfasst, wobei der Gantry und der Personenhalter beweglich ausgestaltet sind.

Die Druckschrift US 2021 /156936 A1 offenbart ein Magnetresonanz-Bildgebungssystem umfassend eine Grundfeldmagnetanordnung mit mehreren räumlich getrennten Magnetsegmenten, deren Segmentfeldrichtungen in einem Winkel zueinander angeordnet sind, sodass ein toroidales Hauptmagnetfeld erzeugt wird.

In der Druckschrift DE 10 2018 212091 A1 wird ein Verfahren, eine Magnetresonanzvorrichtung und ein Computerprogrammprodukt zur Ermittlung einer optimierten MR-Sequenz beschrieben, bei dem anhand des Abstands zwischen einer Lokalspule und einem Körperteil des Patienten sichergestellt wird, dass bei einer MR-Untersuchung ein vorgegebener Belastungsschwellwert, insbesondere der SAR-Wert, nicht überschritten wird.

Es ist daher eine Aufgabe der Erfindung, einen Patientensitz und ein Magnetresonanzgerät bereitzustellen, welche gegenüber konventionellen Systemen eine reduzierte Abmessung aufweisen und eine Vereinfachung eines Arbeitsablaufs einer Patientenpositionierung ermöglichen.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Der erfindungsgemäße Patientensitz ist dazu ausgebildet, einen Patienten während einer Magnetresonanzuntersuchung zu lagern. Vorzugsweise ist der Patientensitz dazu ausgebildet, einen anwendungsgemäß auf dem Patientensitz positionierten Patienten, insbesondere eine diagnostisch relevante Körperregion des Patienten, zu unterstützen und/oder in einer vorbestimmten räumlichen Position zu halten. Es ist ebenso vorstellbar, dass der Patientensitz dazu ausgebildet ist, eine vorbestimmte Körperhaltung des Patienten zumindest während einer Dauer der Magnetresonanzuntersuchung aufrechtzuerhalten.

Der Patientensitz weist einen ersten Abschnitt, einen zweiten Abschnitt, ein Verbindungselement, eine Hochfrequenzeinheit mit zumindest einem Antennenelement, und eine Antriebseinheit auf.

Der erste Abschnitt und der zweite Abschnitt können beliebige Teile des Patientensitzes darstellen. Vorzugsweise ist der erste Abschnitt als eine Sitzfläche des Patientensitzes ausgestaltet. Der zweite Abschnitt kann als eine Lehne des Patientensitzes ausgestaltet sein. Es ist weiterhin vorstellbar, dass der erste Abschnitt oder der zweite Abschnitt als eine Fußstütze oder eine Kopfstütze ausgestaltet ist. Ein als eine Lehne ausgestalteter Abschnitt des Patientensitzes kann weiterhin eine Kopfstütze umfassen. Die Kopfstütze kann in einer unveränderlichen relativen Anordnung zu der Lehne angeordnet oder in die Lehenintegriert sein. Es ist jedoch ebenso vorstellbar, dass die Kopfstütze mittels des Verbindungselements, eines weiteren Verbindungselements oder einer Positionierungseinheit mit einem Abschnitt des Patientensitzes verbunden ist. Die Kopfstütze kann insbesondere einen Abschnitt, z. B. einen dritten Abschnitt oder einen vierten Abschnitt, des Patientensitzes darstellen.

Erfindungsgemäß bilden der erste Abschnitt und der zweite Abschnitt Teile einer Aufnahmefläche für den Patienten. Dies kann bedeuten, dass der erste Abschnitt und der zweite Abschnitt dazu ausgebildet sind, ausgewählte oder zugeordnete Körperregionen eines anwendungsgemäß auf dem Patientensitz positionierten Patienten zu halten oder zu stützen. Vorzugsweise sind der erste Abschnitt und der zweite Abschnitt hierbei in einem direkten Kontakt mit den ausgewählten oder zugeordneten Körperregionen des Patienten.

Das Verbindungselement verbindet den ersten Abschnitt mechanisch mit dem zweiten Abschnitt und ist dazu ausgebildet, eine variable relative Bewegung zwischen dem ersten Abschnitt und dem zweiten Abschnitt zu ermöglichen. Beispielsweise ist das Verbindungselement dazu ausgebildet, die Lehne des Patientensitzes variabel gegenüber der Sitzfläche des Patientensitzes auszurichten.

In einer bevorzugten Ausführungsform sind der erste Abschnitt und der zweite Abschnitt mittels des Verbindungselements unter einem Winkel, insbesondere unter einem von 0° bzw. 180° verschiedenen Winkel, zueinander arrangierbar.

Das Verbindungselement kann ein Gelenk, ein Führungselement, ein Getriebe und/oder ein elastisches Element umfassen oder als ein Gelenk, ein Führungselement, ein Getriebe und/oder ein elastisches Element ausgestaltet sein. Beispielsweise kann das Verbindungselement eine mechanische Feder, ein Winkelgelenk, ein Kugelgelenk, ein Radiallager, ein Axiallager, ein Koppelgetriebe, eine Geradführung, eine Achse und/oder eine Welle umfassen.

Vorzugsweise ist das Verbindungselement dazu ausgebildet, eine relative Positionierung des zweiten Abschnitts zu dem ersten Abschnitt infolge einer manuellen Betätigung durch einen Nutzer (z. B. den Patienten oder ein Mitglied des medizinischen Personals) und/oder einer Betätigung durch ein Positionierungseinheit gemäß einer unten beschriebenen Ausführungsform, zu ermöglichen.

Es ist weiterhin vorstellbar, dass der Patientensitz mehr als zwei Abschnitte umfasst, welche dazu ausgebildet sind, variabel relativ zueinander bewegt zu werden. Vorzugsweise ist das Verbindungselement dazu ausgebildet, die Abschnitte des Patientensitzes, wie z. B. eine Sitzfläche, eine Lehne, eine Kopfstütze und/oder eine Fußstütze, variabel relativ zueinander zu bewegen. Es ist aber ebenso vorstellbar, dass der Patientensitz eine Mehrzahl von Verbindungselementen aufweist, welche dazu ausgebildet sind, die Abschnitte des Patientensitzes variabel relativ zueinander zu bewegen. Beispielsweise kann der Patientensitz ein weiteres Verbindungselement aufweisen, welches dazu ausgebildet sind, eine Kopfstütze variabel relativ zu einer Lehne zu bewegen.

Das zumindest eine Antennenelement der Hochfrequenzeinheit ist dazu ausgebildet, Signale im in einem Leistungs- und Frequenzbereich einer Magnetresonanzuntersuchung zu empfangen.

Das zumindest eine Antennenelement weist vorzugsweise ein oder mehrere Signalleiter auf. Insbesondere kann das zumindest eine Antennenelement ein Koppelelement zwischen in Signalleitern geführten und ungeführten, d. h. in einem Freiraum befindlichen, elektromagnetischen Wellen darstellen. Das zumindest eine Antennenelement ist vorzugsweise dazu ausgebildet, elektromagnetische Wellen im Bereich einer Magnetresonanzfrequenz eines magnetresonanzaktiven Atomkerns zu empfangen. Als hochfrequentes Signal wird dabei beispielsweise eine elektromagnetische Welle mit einer Frequenz zwischen 1 und 500 MHz, vorzugsweise zwischen 10 und 300 MHz angesehen. Das Magnetresonanzsignal üblicher zu untersuchender Atomkerne kann eine geringe Leistung von einigen Mikrowatt bis mehrere Milliwatt aufweisen.

Ein Signalleiter umfasst vorzugsweise einen elektrisch leitenden Draht. Vorzugsweise ist der Draht des Signalleiters dazu ausgebildet, die oben angegebenen Leistungen dauerhaft zu übertragen. Beispielsweise kann der Signalleiter als ein freier Draht, als eine gewickelte Spule oder als eine Leiterbahn auf einer Leiterplatte des zumindest einen Antennenelements ausgeführt sein. Der Signalleiter besteht vorzugsweise aus Kupfer. Es sind aber auch andere elektrisch leitende Metalle, wie z. B. Gold, Silber oder Aluminium, vorstellbar.

Das zumindest eine Antennenelement kann einen oder mehrere spulenförmige Signalleiter aufweisen. Es ist vorstellbar, dass das zumindest eine Antennenelement eine Schmetterlings-Spule (*butterfly coil*) umfasst, welche entlang einer Anatomie des Patienten, insbesondere einer Kiefergelenksregion, faltbar oder schwenkbar ist.

In einer bevorzugten Ausführungsform des Patientensitzes ist die Hochfrequenzeinheit bei anwendungsgemäßer Positionierung des Patienten auf dem Patientensitz an einer Kopfregion des Patienten angeordnet. Die Hochfrequenzeinheit kann als eine Kopfspule, insbesondere als eine Dentalspule, ausgestaltet sein. Die zumindest eine Antenneneinheit der Hochfrequenzeinheit kann als eine Empfangsspule und/oder eine Sendespule (Transmissionsspule) ausgestaltet sein. Es ist vorstellbar, dass die Hochfrequenzeinheit eine Mehrheit von Antennenelementen, insbesondere zumindest eine Empfangsspule und zumindest eine Sendespule, umfasst.

In einer besonders bevorzugten Ausführungsform ist die Hochfrequenzeinheit permanent oder irreversibel in einen Abschnitt des Patientensitzes, insbesondere die Kopfstütze oder die Lehne, integriert. Es ist vorstellbar, dass die Hochfrequenzeinheit des Patientensitzes einen Teil eines Hochfrequenzsystems eines Magnetresonanzgeräts bildet und/oder von einer Hochfrequenzsteuereinheit eines Magnetresonanzgeräts ansteuerbar ausgestaltet ist.

Die Antriebseinheit ist dazu ausgebildet ist, den Patientensitz variabel entlang einer Raumrichtung zu bewegen. Vorzugsweise ist die Antriebseinheit dazu ausgebildet, den Patientensitz entlang einer Patientenzugangsrichtung eines Magnetresonanzgeräts zu bewegen. Die Patientenzugangsrichtung kann mit einer Längsrichtung eines Patientenaufnahmebereichs des Magnetresonanzgeräts (z. B. einer Z-Richtung) übereinstimmen oder parallel zu der Längsrichtung eines Patientenaufnahmebereichs des Magnetresonanzgeräts ausgerichtet sein. Die Raumrichtung, aber auch die Patientenzugangsrichtung des Magnetresonanzgeräts, können von einer Horizontalen abweichen oder unter einem von Null Grad (und/oder 180 Grad) verschiedenen Winkel, z. B. einem Winkel zwischen 1 und 70°, vorzugsweise einem Winkel zwischen 5 und 45°, zu einer Horizontalen ausgerichtet sein.

Die Antriebseinheit kann einen beliebigen Antrieb, beispielsweise einen elektrischen oder pneumatischen Antrieb, insbesondere einen hydraulischen Antrieb, umfassen. Es ist weiterhin vorstellbar, dass die Antriebseinheit ein Führungselement, wie z. B. ein Getriebe, ein Lager, eine Schiene, eine Führungsstange, eine Achse, eine Welle und/oder eine Linearführung, umfasst, welches dazu ausgebildet ist, den Patientensitz mechanisch mit der Antriebseinheit zu koppeln. Die Antriebseinheit kann entsprechend dazu ausgebildet sein, den Patientensitz entlang einer von dem Führungselement und/oder der Antriebseinheit vorgegebenen Bewegungstrajektorie zu bewegen.

Der erfindungsgemäße Patientensitz, ermöglicht auf vorteilhafte Weise eine Reduktion einer Abmessung gegenüber konventionellen Patientenlagerungsvorrichtungen für den Einsatz in der Magnetresonanztomographie. Beispielsweise kann der erfindungsgemäße Patientensitz eine Magnetresonanzuntersuchung eines aufrecht-sitzenden Patienten oder eines zurückgelehnten Patienten mit einem dedizierten Magnetresonanzgerät ermöglichen, wodurch sich Standortanforderungen, wie z. B. eine Größe eines Untersuchungsraums, eine Tragfähigkeit eines Bodens und/oder eine Abmessung eines Zugangswegs zu dem Untersuchungsraum, auf vorteilhafte Weise mildern lassen. Weiterhin ermöglicht eine erfindungsgemäße Unterteilung des Patientensitzes in zumindest den ersten Abschnitt und den zweiten Abschnitt eine gewinkelte Anordnung von Körperregionen oder Extremitäten relativ zu einem Oberkörper des Patienten. Dadurch können Muskeln, Gelenke, aber auch die Wirbelsäule des Patienten, vorteilhaft von Druck entlastet werden, wodurch ein Komfort des Patienten während der Magnetresonanzuntersuchung erhöht und/oder ein Risiko eines Abbruchs der Magnetresonanzuntersuchung reduziert werden können.

Durch eine in den Patientensitz integrierte Hochfrequenzeinheit lassen sich mit der Anordnung oder Anbindung der Hochfrequenzeinheit verbundene Arbeitsschritte vermeiden, wodurch die Effizienz einer Patientenvorbereitung für die Magnetresonanzuntersuchung vorteilhaft erhöht wird. Weiterhin lassen sich durch die Integration der Hochfrequenzeinheit in den Patientensitz lose elektrische Kabel und/oder andere Teile Komponenten der Hochfrequenzeinheit in der Nähe des Patienten vermeiden, wodurch ein Irritieren des Patienten, aber auch ein Verschleiß mechanischer und elektrischer Teile der Hochfrequenzeinheit durch Kontakt mit dem Patienten, auf vorteilhafte Weise vermieden oder reduziert werden können.

Der erfindungsgemäße Patientensitz kann dazu ausgebildet sein, den Patienten in einer aufrecht-sitzenden Haltung aufzunehmen. Es ist vorstellbar, dass die Haltung des Patienten mittels des Verbindungselements und/oder einer Positionierungseinheit manuell, automatisch oder teilweise automatisiert einstellbar ist, um den Patienten aus der aufrecht-sitzenden in eine für die Magnetresonanzuntersuchung geeignete Haltung zu überführen.

Ein erfindungsgemäßer Patientensitz ermöglicht ein selbstständiges Positionieren des Patienten ohne weitere Anpassung Korrektur oder Unterstützung durch medizinisches Personal. Insbesondere kann ein Patientensitz mit zwei unter einem Winkel zueinander ausgerichteten Abschnitten eine anwendungsgemäße Positionierung des Patienten definieren. Eine weitere Anpassung einer Haltung des Patienten für die Durchführung der Magnetresonanzuntersuchung kann mittels einer relativen Anordnung des ersten Abschnitts und des zweiten Abschnitts vorgenommen werden, wodurch eine aufwändige Patientenpositionierung vorteilhaft vermieden wird.

In einer bevorzugten Ausführungsform ist der erfindungsgemäße Patientensitz bzw. seine Komponenten, wie z.B. der erste Abschnitt, der zweite Abschnitt, das Verbindungselement, eine Positionierungseinheit und/oder die Antriebseinheit, aus einem Material gefertigt, welches mit einer Magnetresonanzuntersuchung kompatibel ist. Dies kann bedeuten, dass das Material eine Störung der Magnetresonanzuntersuchung, insbesondere ein Auftreten von Bildartefakten, vermeidet.

In einer Ausführungsform weist der erfindungsgemäße Patientensitz eine Positionierungseinheit auf, welche dazu ausgebildet ist, den ersten Abschnitt automatisch variabel relativ zu dem zweiten Abschnitt zu positionieren.

Die Positionierungseinheit kann ein Teil des Verbindungselements darstellen oder dieses umfassen. Es ist vorstellbar, dass die Positionierungseinheit einen Antrieb aufweist, welcher dazu ausgebildet ist, den ersten Abschnitt variabel relativ zu dem zweiten Abschnitt zu positionieren. Der Antrieb der Positionierungseinheit kann als ein elektrischer Antrieb, ein pneumatischer Antrieb oder ein hydraulischer Antrieb ausgestaltet sein. Es ist weiterhin vorstellbar, dass die Positionierungseinheit dazu ausgebildet ist, eine von dem Antrieb bereitgestellte Energie zu speichern. Beispielsweise kann die Positionierungseinheit eine mechanische Feder oder eine Gasdruckfeder umfassen, welche dazu ausgebildet ist, eine von dem Antrieb bereitgestellte Energie zu speichern und kontrolliert in eine Änderung einer relativen Anordnung des ersten Abschnitts zu dem zweiten Abschnitt umzuwandeln.

Vorzugsweise weisen das Verbindungselement und/oder die Positionierungseinheit zumindest ein Sicherungselement auf, welches dazu ausgebildet ist, eine relative Bewegung des ersten Abschnitts zu dem zweiten Abschnitt zu begrenzen. Beispielsweise kann das Sicherungselement als ein Anschlagselement ausgestaltet sein. Ein Sicherungselement kann dazu ausgebildet sind, einen Bewegungsspielraum zwischen dem ersten Abschnitt und dem zweiten Abschnitt festzulegen oder zu begrenzen. Es ist insbesondere vorstellbar, dass ein Führungselement des Verbindungselements und/oder der Positionierungseinheit ein Sicherungselement aufweist, welches einen Drehwinkel und/oder einen Kippwinkel des zweiten Abschnitts relativ zu dem ersten Abschnitt einschränkt.

Mittels einer erfindungsgemäßen Positionierungseinheit lässt sich eine Körperhaltung eines auf dem Patientensitz angeordneten Patienten auf vorteilhafte Weise zeiteffizient in Abhängigkeit einer zu untersuchenden Körperregion anpassen.

In einer Ausführungsform des erfindungsgemäßen Patientensitzes ist die Antriebseinheit dazu ausgebildet, den Patientensitz variabel entlang einer ersten Raumrichtung und einer zweiten Raumrichtung, welche orthogonal zu der ersten Raumrichtung ausgerichtet ist, zu positionieren.

Die erste Raumrichtung kann beispielsweise mit einer Patientenzugangsrichtung und/oder einer Z-Richtung eines Magnetresonanzgeräts übereinstimmen. Die zweite Raumrichtung kann dagegen mit einer Y-Richtung eines Magnetresonanzgeräts und/oder einer anterior-posterior-Richtung eines Oberkörpers eines anwendungsgemäß auf dem Patientensitz positionierten Patienten übereinstimmen.

In einer Ausführungsform ist die Antriebseinheit dazu ausgebildet, den Patientensitz variabel entlang einer drittem Raumrichtung, welche orthogonal zu der ersten Raumrichtung und der zweiten Raumrichtung ausgerichtet ist, zu positionieren.

In einer bevorzugten Ausführungsform weicht die erste Raumrichtung von einer gedachten Horizontalen ab. Beispielsweise kann eine Längsachse eines Patientenaufnahmebereichs des Magnetresonanzgeräts eine Neigung gegenüber der gedachten Horizontalen aufweisen, sodass auch die Z-Richtung oder die Patientenzugangsrichtung des Magnetresonanzgeräts einen Winkel oder eine Neigung gegenüber der gedachten Horizontalen aufweisen. Die Antriebseinheit kann somit insbesondere dazu ausgebildet sein, den Patientensitz variabel entlang einer Trajektorie zu bewegen, welche einen von Null verschiedenen Winkel zu der gedachten Horizontalen aufweist.

Mittels des erfindungsgemäßen Patientensitzes lässt sich eine räumliche Position einer diagnostisch relevanten Körperregion eines anwendungsgemäß auf dem Patientensitz positionierten Patienten entlang mehrerer Raumrichtungen automatisch mit einer räumlichen Position eines Bildgebungsvolumens eines Magnetresonanzgeräts abstimmen. Dadurch kann eine Unterstützung der Patientenpositionierung durch das medizinische Personal reduziert oder vollständig vermieden werden. Weiterhin lassen sich diagnostisch relevante Körperregionen mittels des erfindungsgemäßen Patientensitzes auf vorteilhafte Weise mit hoher Genauigkeit positionieren, wodurch auch Magnetresonanzgeräte mit kleinen Bildgebungsvolumen und einem geringen Flächenbedarf verwendet werden können.

Ein Bildgebungsvolumen kann durch eine vorbestimmte Magnetfeldrichtung und/oder eine vorbestimmte magnetische Feldstärke charakterisiert sein. Zum Beispiel kann das Bildgebungsvolumen ein Volumen mit einer im Wesentlichen gleichmäßigen Magnetfeldrichtung und/oder einer im Wesentlichen homogenen magnetischen Feldstärke umfassen. Ein Bildgebungsvolumen kann mit einem Isozentrum eines Magnetresonanzgeräts übereinstimmen.

Weiterhin lässt sich durch eine geneigte Patientenzugangsrichtung und eine Antriebseinheit, welche dazu ausgebildet ist, den Patientensitz unter einem Winkel zu einer gedachten Horizontalen zu bewegen, ein Flächenbedarf des Patientensitzes und/oder des Magnetresonanzgeräts vorteilhaft reduzieren.

In einer bevorzugten Ausführungsform weist der erfindungsgemäße Patientensitz eine Kopfstütze und ein weiteres Verbindungselement auf.

Die Kopfstütze kann mittels des weiteren Verbindungselements mit dem ersten Abschnitt und/oder dem zweiten Abschnitt des Patientensitzes mechanisch gekoppelt oder verbunden sein.

Es ist vorstellbar, dass das weitere Verbindungselement als ein Teil einer Positionierungseinheit oder einer weiteren Positionierungseinheit gemäß einer hierin beschriebenen Ausführungsform ausgestaltet ist. Das weitere Verbindungselement kann die weitere Positionierungseinheit auch umfassen.

Das weitere Verbindungselement kann entsprechend einer Ausführungsform des Verbindungselements ausgestaltet sein. Erfindungsgemäß ist das weitere Verbindungselement dazu ausgebildet, die Kopfstütze variabel relativ zu dem ersten Abschnitt und/oder dem zweiten Abschnitt zu positionieren, wobei die Hochfrequenzeinheit an der Kopfstütze angeordnet ist.

Die Kopfstütze kann dazu ausgebildet sein, den Kopf oder eine räumliche Position des Kopfes eines anwendungsgemäß auf dem Patientensitz positionierten Patienten zu stützen oder zu stabilisieren.

Vorzugsweise ist das weitere Verbindungselement dazu ausgebildet, eine räumliche Position der Kopfstütze automatisch relativ zu einer räumlichen Position des zweiten Abschnitts und/oder des ersten Abschnitts zu ändern. Beispielsweise ist das weitere Verbindungselement dazu ausgebildet, die Kopfstütze relativ zu einer Lehne des Patientensitzes zu bewegen.

Mittels einer erfindungsgemäßen Kopfstütze lässt sich eine räumliche Position und/oder Ausrichtung des Kopfes eines anwendungsgemäß auf dem Patientensitz positionierten Patienten vorteilhaft anpassen. Dadurch kann der Kopf eines Patienten unabhängig von anderen Körperregionen des Patienten räumlich positioniert und/oder ausgerichtet werden, um eine räumliche Position einer diagnostisch relevanten Region des Kopfes mit einer räumlichen Position eines Bildgebungsvolumens eines Magnetresonanzgeräts abzustimmen. Im Gegensatz zu konventionellen Patientenlagerungsvorrichtungen lässt sich eine aufwändige Positionierung des gesamten Körpers des Patienten somit auf vorteilhafte Weise vermeiden.

Ferner lässt sich durch eine automatische Positionierung des Kopfes des Patienten mittels der Kopfstütze des erfindungsgemäßen Patientensitzes im Vergleich zu einer manuellen Positionierung eine genauere räumliche Positionierung einer diagnostisch relevanten Körperregion erreichen. Dadurch können auf vorteilhafte Weise Magnetresonanzgeräte mit geringerer magnetischer Feldstärke und/oder kleineren Bildgebungsvolumina verwendet werden, welche einen geringeren Platzbedarf erfordern.

In einer Ausführungsform ist die Hochfrequenzeinheit an der Kopfstütze des Patientensitzes angeordnet. Beispielsweise ist die Hochfrequenzeinheit mechanisch mit der Kopfstütze verbunden oder in die Kopfstütze integriert.

Durch eine Anordnung der Hochfrequenzeinheit an der Kopfstütze kann das zumindest eine Antennenelement von der Kopfstütze gehalten und mittels eines Verbindungselements und/oder einer Positionierungseinheit gemäß einer hierin beschriebenen Ausführungsform zusammen mit der Kopfstütze räumlich positioniert und/oder ausgerichtet werden. Dadurch lässt sich eine Bereitstellung eines separaten Mechanismus zur Einstellung einer relativen Position zwischen der Hochfrequenzeinheit und dem Patientensitz vorteilhaft vermeiden. Insbesondere erlaubt die Anordnung der Hochfrequenzeinheit an der Kopfstütze eine Einhaltung einer vorbestimmten Position zwischen dem Kopf eines anwendungsgemäß auf dem Patientensitz positionierten Patienten und der zumindest einen Antenneneinheit. Dadurch lassen sich Fehler bei einer manuellen Positionierung der Hochfrequenzeinheit an einer diagnostisch relevanten Region des Patienten auf vorteilhafte Weise vermeiden.

In einer weiteren Ausführungsform des erfindungsgemäßen Patientensitzes ist das weitere Verbindungselement dazu ausgebildet, eine variable Positionierung der Kopfstütze im Wesentlichen parallel zu einer anterior-posterior-Richtung und/oder einer superior-inferior-Richtung eines Oberkörpers eines anwendungsgemäß auf dem Patientensitz positionierten Patienten zu ermöglichen.

Eine anterior-posterior-Richtung eines Oberkörpers eines anwendungsgemäß auf dem Patientensitz positionierten Patienten kann mit einer Y-Richtung eines für die Magnetresonanzuntersuchung verwendeten Magnetresonanzgeräts übereinstimmen. Eine superior-inferior-Richtung eines Oberkörpers eines anwendungsgemäß auf dem Patientensitz positionierten Patienten kann hingegen mit einer Z-Richtung eines für die Magnetresonanzuntersuchung verwendeten Magnetresonanzgeräts übereinstimmen. Es ist vorstellbar, dass die anterior-posterior-Richtung des Oberkörpers des Patienten um einen Winkel von bis zu 15°, bis zu 30° oder bis zu 45°, von der Y-Richtung des verwendeten Magnetresonanzgeräts abweicht. Ebenso kann die superior-inferior-Richtung des Oberkörpers des Patienten um einen Winkel von bis zu 15°, bis zu 30° oder bis zu 45°, von der Z-Richtung des verwendeten Magnetresonanzgeräts abweichen.

In einer Ausführungsform ist das weitere Verbindungselement dazu ausgebildet, die Kopfstütze um eine bei anwendungsgemäßer Positionierung des Patienten auf dem Patientensitz im Wesentlichen zu einer medialen Richtung des Patienten parallel ausgerichteten Achse zu rotieren oder zu schwenken.

Eine variable Positionierung der Kopfstütze ermöglicht eine besonders zeiteffiziente Abstimmung einer räumlichen Position einer großen Anzahl diagnostisch relevanter Regionen des Kopfes mit einer räumlichen Position eines Bildgebungsvolumens eines Magnetresonanzgeräts mit einem geringen technischen Aufwand.

In einer Ausführungsform des erfindungsgemäßen Patientensitzes ist das Verbindungselement dazu ausgebildet, den ersten Abschnitt derart variabel relativ zu dem zweiten Abschnitt zu bewegen, dass eine relative Position des Kopfes eines anwendungsgemäß auf dem Patientensitz positionierten Patienten und eines Abschnitts des Patientensitzes dabei im Wesentlichen unverändert bleibt.

Das Verbindungselement kann insbesondere dazu ausgebildet sein, den ersten Abschnitt derart variabel relativ zu dem zweiten Abschnitt zu bewegen, dass eine relative Position des Kopfes eines anwendungsgemäß auf dem Patientensitz positionierten Patienten und eines den Kopf des Patienten stützenden Abschnitts, beispielsweise einer Kopfstütze und/oder einer Lehne, des Patientensitzes dabei im Wesentlichen unverändert bleibt.

Es ist vorstellbar, dass das Verbindungselement und/oder die Positionierungseinheit dazu ausgebildet sind, eine relative räumliche Anordnung des ersten Abschnitts und des zweiten Abschnitts in Abhängigkeit eines anatomisch korrekten Bewegungsmusters, insbesondere eines Modells, einer menschlichen Person zu verändern. Beispielsweise kann das Verbindungselement dazu ausgebildet sein, eine Lehne derart relativ zu einer Sitzfläche des Patientensitzes zu bewegen, dass sich eine relative Position zwischen einem Rückenabschnitt eines Patienten, welcher sich anwendungsgemäß auf dem Patientensitz zurücklehnt, und der Lehne im Wesentlichen unverändert bleibt.

Weiterhin können das Verbindungselement und/oder das weitere Verbindungselement dazu ausgebildet sein, eine Kopfstütze derart relativ zu einer Sitzfläche des Patientensitzes zu bewegen, dass eine relative Position zwischen dem Kopf des Patienten, welcher sich anwendungsgemäß auf dem Patientensitz zurücklehnt, und der Kopfstütze im Wesentlichen unverändert bleibt.

Das Verbindungselement und/oder das weitere Verbindungselement können ein Koppelgetriebe oder eine Mehrzahl von Führungselementen, beispielsweise ein Gelenk und ein Gleitlager, eine Mehrzahl von Gelenken, oder ein oder mehrere Gelenke und Lager aufweisen. Das Koppelgetriebe oder die Mehrzahl von Führungselementen können dazu ausgebildet sein, einen Abschnitt des Patientensitzes entlang einer elliptischen Bewegungstrajektorie relativ zu einem weiteren Abschnitt des Patientensitzes zu bewegen. Beispielsweise können das Verbindungselement und/oder das weitere Verbindungselement dazu ausgebildet sein, die Lehne bei einer Rotation gegenüber der Sitzfläche in vertikaler Richtung zu verschieben. Auf diese Weise kann eine relative Bewegung zwischen der Lehne und einem Rückenabschnitt des Patienten, aber auch dem Kopf und der Kopfstütze, vermieden werden.

Es ist ebenso vorstellbar, dass die Positionierungseinheit und/oder die weitere Positionierungseinheit dazu ausgebildet sind, eine relative räumliche Anordnung des ersten Abschnitts und des zweiten Abschnitts, aber auch eine relative räumliche Anordnung eines dritten Abschnitts und des zweiten Abschnitts, mittels des Verbindungselements und/oder des weiteren Verbindungselements in Abhängigkeit eines anatomisch korrekten Bewegungsmusters oder Modells einer menschlichen Person zu verändern. Ein solches Bewegungsmuster oder Modell einer menschlichen Person kann beispielsweise aus einer Datenbank eingelesen und mittels einer Recheneinheit verarbeitet werden. Eine Steuereinheit kann dazu ausgebildet sein, auf Basis des Bewegungsmusters oder Modells der menschlichen Person einen Steuerbefehl auszugeben, welcher die Positionierungseinheit und/oder die weitere Positionierungseinheit ansteuert. Die Recheneinheit und die Steuereinheit können in den Patientensitz oder ein Magnetresonanzgerät integriert oder als eigenständige Komponenten ausgestaltet sein.

Ein erfindungsgemäßer Patientensitz ermöglicht eine selbstständige Positionierung eines Patienten auf dem Patientensitz durch Einnahme einer Sitzposition. Durch Vermeidung einer relativen Bewegung zwischen Abschnitten des Patientensitzes und mit den Abschnitten korrespondierenden Körperregionen des Patienten lässt sich eine Anleitung des Patienten durch Mitglieder des medizinischen Personals vermeiden und ein Arbeitsaufwand bei der Patientenpositionierung reduzieren.

Weiterhin können Teile einer Hochfrequenzeinheit, insbesondere das zumindest eine Antennenelement, bereits an einem aufrecht in dem Patientensitz positionierten Patienten angeordnet werden. Dadurch lässt sich ein Vorgang einer Anordnung eines Teils einer Hochfrequenzeinheit relativ zu dem Patienten auf vorteilhafte Weise vereinfachen. Insbesondere kann ein Mitglied des medizinischen Personals bei Anordnung des zumindest einen Antennenelements an einem aufrecht sitzenden Patienten im Vergleich zu einem liegenden Patienten eine ergonomischere Körperhaltung einnehmen, wodurch sich ein Auftreten von Haltungsschäden oder haltungsbedingten Krankheitsbildern vermeiden oder reduzieren lässt.

In einer weiteren Ausführungsform weist die Hochfrequenzeinheit des erfindungsgemäßen Patientensitzes ein Führungselement auf, welches dazu ausgebildet ist, das zumindest eine Antennenelement variabel relativ zu einem Abschnitt des Patientensitzes zu bewegen.

Ein Führungselement kann nach einer oben beschriebenen Ausführungsform ausgestaltet sein. Insbesondere kann das Führungselement Drehlager, ein Gleitlager, ein Gelenk, ein Scharnier und/oder ein Faltelement umfassen. Vorzugsweise ist das Führungselement dazu ausgebildet, zumindest einen Abschnitt des zumindest einen Antennenelements um einen durch das Führungselement definierten Achspunkt zu bewegen oder zu rotieren. Das Führungselement kann an einem Ende des zumindest einen Antennenelements angeordnet sein oder das zumindest eine Antennenelement in mehrere Abschnitte unterteilen.

Es ist vorstellbar, dass die Hochfrequenzeinheit ein oder mehrere Gelenke aufweist, welche dazu ausgebildet sind, mehrere Abschnitte des zumindest einen Antennenelements variabel relativ zueinander zu bewegen. Die ein oder mehreren Gelenke können weiterhin dazu ausgebildet sein, das zumindest eine Antennenelement oder die mehreren Abschnitte des zumindest einen Antennenelements variabel relativ zu der Hochfrequenzeinheit und/oder dem zweiten Abschnitt des Patientensitzes zu bewegen.

In einer bevorzugten Ausführungsform ist das Führungselement als ein Axiallager oder ein Scharnier ausgestaltet, welches dazu ausgebildet ist, das zumindest eine Antennenelement um eine parallel zu einer Sagittalebene eines Oberkörpers, insbesondere des Kopfes, eines anwendungsgemäß auf dem Patientensitz positionierten Patienten ausgerichteten Achse zu schwenken oder zu rotieren.

In einer weiteren Ausführungsform weist die Hochfrequenzeinheit zumindest ein zweites Antennenelement und ein weiteres Führungselement nach einer oben beschriebenen Ausführungsform auf. Vorzugsweise sind das zumindest eine Antennenelement und das zweite Antennenelement derart an dem Patientensitz angeordnet oder befestigt, dass sie einen anwendungsgemäß auf dem Patientensitz positionierten Patienten von zwei gegenüberliegenden Seiten flankieren.

In einer Ausführungsform ist das Führungselement dazu ausgebildet, das zumindest eine Antennenelement in diskreten Schritten oder Abständen relativ zu einem Abschnitt des Patientensitzes zu bewegen. Das Führungselement kann hierfür beispielweise einen Rastmechanismus und/oder ein Rastelement aufweisen.

Eine erfindungsgemäße Hochfrequenzeinheit mit einem Führungselement erlaubt eine besonders zeiteffiziente Anordnung des zumindest einen Antennenelements an einem auf dem Patientensitz angeordneten Patienten. Insbesondere kann mittels einer relativen Anordnung des zumindest einen Antennenelements zu dem Patienten in diskreten Schritten oder Abständen eine effiziente Anpassung an eine Größe einer Körperregion eines Patienten erfolgen. Weiterhin lässt sich mit einem Antennenelement mit einem erfindungsgemäßen Führungsmechanismus ein verbessertes oder optimiertes Signal-zu-Rausch Verhältnis bereitstellen.

In einer weiteren Ausführungsform des erfindungsgemäßen Patientensitzes weist die Hochfrequenzeinheit einen Schwenkmechanismus auf, welcher dazu ausgebildet ist, das zumindest eine Antennenelement um eine bei anwendungsgemäßer Positionierung des Patienten auf dem Patientensitz im Wesentlichen parallel zu einer medialen Richtung des Patienten ausgerichteten Achse zu schwenken.

Ein Schwenkmechanismus kann ein Führungselement nach einer oben beschriebenen Ausführungsform umfassen. Vorzugsweise weist der Schwenkmechanismus zumindest ein Drehlager, insbesondere ein Axiallager, oder ein Scharnier auf.

Der Schwenkmechanismus kann eine Schwenkachse oder eine Rotationsachse definieren, welche im Wesentlichen parallel zu einer medialen Richtung oder einer lateralen Richtung, insbesondere einer Transversalebene, des Oberkörpers oder des Kopfes eines anwendungsgemäß auf dem Patientensitz positionierten Patienten ausgerichtet ist.

Es ist vorstellbar, dass der Schwenkmechanismus dazu ausgebildet ist, das zumindest eine Antennenelement an einer Seite des Patienten um besagte Rotationsachse zu schwenken. Vorzugsweise weist die Hochfrequenzeinheit zumindest ein weiteres Antennenelement auf. Das zumindest eine Antennenelement und das weitere Antennenelement können derart mittels des Schwenkmechanismus an dem Patientensitz gehalten werden, dass sie einen anwendungsgemäß auf dem Patientensitz positionierten Patienten von zwei gegenüberliegenden Seiten flankieren. Es ist weiterhin vorstellbar, dass das zumindest eine Antennenelement und das weitere Antennenelement mittels des Schwenkmechanismus gekoppelt sind und synchron zueinander um die Schwenkachse des Schwenkmechanismus schwenkbar sind.

Eine erfindungsgemäße Hochfrequenzeinheit mit einem Schwenkmechanismus ermöglicht ein Anordnen von mehreren Antennenelementen in einer vorbestimmten relativen Position zu dem Patienten von einer Seite des Patientensitzes. Dadurch kann ein Arbeitsschritt eines gentrennten Positionierens von Antennenelementen auf vorteilhafte Weise vermieden werden.

Weiterhin ermöglichen an dem Patientensitz gefestigte Antennenelemente gegenüber konventionellen Auflegespulen und abnehmbaren Lokalspulen eine Reduktion zumindest eines Bewegungsfreiheitsgrades, wodurch ein Arbeitsschritt eines Positionierens und/oder Ausrichtens des zumindest einen Antennenelements mit reduziertem Zeitaufwand durchgeführt werden kann.

Die hierin beschriebenen Ausführungsformen der Hochfrequenzeinheit können eine Anordnung des zumindest einen Antennenelements an einem Patienten ohne besondere Fachkenntnisse ermöglichen, wodurch eine aufwendige Schulung von medizinischem Personal vorteilhaft vermieden werden kann.

Das erfindungsgemäße Magnetresonanzgerät ist zum Durchführen einer Magnetresonanzuntersuchung eines in einem Patientenaufnahmebereich des Magnetresonanzgeräts angeordneten Patienten geeignet.

Vorzugsweise ist das Magnetresonanzgerät dazu ausgebildet, eine Magnetresonanzuntersuchung eines innerhalb eines Bildaufnahmebereichs des Magnetresonanzgeräts positionierten Patienten, insbesondere eines anwendungsgemäß auf einem erfindungsgemäßen Patientensitz angeordneten Patienten, durchzuführen. Der Bildaufnahmebereich kann im Wesentlichen mit dem Patientenaufnahmebereich übereinstimmen oder einen Teil des Patientenaufnahmebereichs bilden. Vorzugsweise ist das Magnetresonanzgerät dazu ausgebildet, Magnetresonanzdaten oder Magnetresonanzsignale von dem Patienten zu erfassen. Ferner kann das Magnetresonanzgerät dazu ausgebildet sein, Magnetresonanzbilddaten, insbesondere diagnostische Magnetresonanzbilddaten, von dem innerhalb des Bildaufnahmebereichs positionierten Patienten zu erfassen.

Das Magnetresonanzgerät kann ein Gradientensystem mit einer oder mehreren Gradientenspulen umfassen. Weiterhin kann das Magnetresonanzgerät eine Hochfrequenzspule, insbesondere eine fest in das Magnetresonanzgerät integrierte Körperspule, umfassen. In einer bevorzugten Ausführungsform weisen die Gradientenspule(n) und die Hochfrequenzspule elektrische Leiterstrukturen auf, welche schalenförmig oder zylinderförmig ausgestaltet sind und den Bildaufnahmebereich des Magnetresonanzgeräts entlang einer Patientenzugangsrichtung umschließen. Es ist vorstellbar, dass die Gradientenspule(n) die Hochfrequenzspule entlang der Patientenzugangsrichtung umschließen.

In einer bevorzugten Ausführungsform ist die Patientenzugangsrichtung des Magnetresonanzgeräts unter einem von Null Grad verschiedenen Winkel zu einer gedachten Horizontalen angeordnet. Es ist vorstellbar, dass eine von dem Patientenaufnahmebereich des Magnetresonanzgeräts definierte Längsachse in Richtung einer im Wesentlichen horizontalen Bodenfläche eines Untersuchungsraums, in welchem das Magnetresonanzgerät installiert ist, geneigt ist. Beispielsweise kann ein Winkel zwischen einer Orthogonalen zu der Bodenfläche und der Patientenzugangsrichtung weniger als 90°, vorzugsweise weniger als 80°, besonders bevorzugt weniger als 70°, betragen.

In einer bevorzugen Ausführungsform ist das erfindungsgemäße Magnetresonanzgerät als ein geschlossener Scanner ("*closed-bore scanner*") oder ein Scanner mit einem zylindrisch geformten Patientenaufnahmebereich ausgestaltet. Ein geschlossener Scanner kann einen im Wesentlichen zylindrisch geformten Bildaufnahmebereich aufweisen. Ein Hauptmagnet des geschlossenen Scanners kann eine oder mehrere Magnetspulen umfassen, welche den Bildaufnahmebereich entlang einer axialen Richtung oder einer Rotationsachse, insbesondere einer Rotationssymmetrie-Achse, des Hauptmagneten umschließen. Eine Magnetspule kann einen elektrischen Leiter mit vernachlässigbarem elektrischem Widerstand bei (oder unter) einer supraleitenden Temperatur umfassen. Eine Richtung eines Hauptmagnetfeldes, welches mittels des Hauptmagneten bereitgestellt wird, kann im Wesentlichen parallel zu der Patientenzugangsrichtung und/oder der axialen Richtung des Patientenaufnahmebereichs ausgerichtet sein.

Es ist ebenso vorstellbar, dass das erfindungsgemäße Magnetresonanzgerät als ein offener Scanner ("open-bore scanner") ausgestaltet ist. Ein Hauptmagnet eines offenen Scanners kann zwei Magnete umfassen, welche sich durch den Bildaufnahmebereich getrennt gegenüberstehen. Eine Richtung des Hauptmagnetfeldes des offenen Scanners kann im Wesentlichen orthogonal zu einer Patientenzugangsrichtung zu dem Bildaufnahmebereich und/oder einer Längsrichtung Richtung des Bildaufnahmebereichs ausgerichtet sein.

Der Hauptmagnet des Magnetresonanzgeräts kann einen oder mehrere Elektromagnete oder supraleitende Magnete umfassen oder aus solchen bestehen. In einer bevorzugten Ausführungsform umfasst oder besteht der Hauptmagnet aus einem oder mehreren zylindrisch geformten supraleitenden Magneten bzw. supraleitenden Spulen. Der Hauptmagnet kann mechanisch mit einer Magnethaltestruktur gekoppelt und/oder an der Magnethaltestruktur befestigt sein. Vorzugsweise ist die Magnethaltestruktur dazu ausgebildet, den Hauptmagneten zu tragen und/oder zu stützen. Der Begriff "Hauptmagnet" kann eine oder mehrere Magnete oder Spulen sowie eine dedizierte Stützstruktur für die Magnete oder Spulen umfassen.

In einer bevorzugten Ausführungsform ist das erfindungsgemäße Magnetresonanzgerät als ein "trockenes" System ausgestaltet. Ein "trockenes" System kann eine geringe Menge an Kryogen oder gar kein Kryogen enthalten. Zum Beispiel kann das erfindungsgemäße Magnetresonanzgerät einen oder mehrere kleine Kryogenbehälter umfassen, welche mittels einer Wärmeleitstruktur thermisch mit dem Hauptmagneten verbunden sind. Ein Kryogenbehälter eines "trockenen" Systems kann ein Volumen von weniger als 10 l, weniger als 5 l oder weniger als 1 l an Kryogen enthalten. In einer Ausführungsform wird auf Kryogenbehälter verzichtet. In diesem Fall wird der Hauptmagnet vollständig mittels einer Wärmeleitstruktur gekühlt. Durch Verwendung eines "trockenen" Systems lassen sich ein Gewicht des Magnetresonanzgeräts reduzieren, aber auch eine mit einem Entweichen von Kryogen im Falle eines Quench verbundene Infrastruktur (z. B. eine sogenannte Quenchpipe) vermeiden. Dadurch lassen sich ein Raumbedarf, aber auch weitere Standortanforderungen, des erfindungsgemäßen Magnetresonanzgeräts auf vorteilhafte Weise reduzieren.

In einer alternativen Ausführungsform ist das Magnetresonanzgerät als ein "nasses" System ausgestaltet. Ein "nasses" System kann zumindest einen Kryogenbehälter mit einem Volumen von mehr als 10 l umfassen. Vorzugsweise ist der Hauptmagnet bei "nassen" Systemen innerhalb des Kryogenbehälters angeordnet und direkt durch das Kryogen gekühlt.

Ein Kryogen kann ein Fluid mit einem niedrigen Siedepunkt, wie z. B. Argon, Stickstoff, Neon, Helium oder dergleichen, darstellen. Eine Kühltemperatur des Kryogens kann im Wesentlichen einer supraleitenden Temperatur des Hauptmagneten entsprechen.

Die hierin erfindungsgemäßen Konzepte lassen sich auch auf Magnetresonanzgeräte mit Hauptmagneten übertragen, welche Permanentmagnete umfassen oder aus Permanentmagneten bestehen.

Das Magnetresonanzgerät weist einen Patientensitz nach einer oben beschriebenen Ausführungsform auf.

Erfindungsgemäß ist die Antriebseinheit dazu ausgebildet, den Patientensitz zumindest entlang einer Raumrichtung variabel relativ zu dem Patientenaufnahmebereich des Magnetresonanzgeräts zu bewegen.

Vorzugsweise ist die Antriebseinheit dazu ausgebildet, den Patientensitz variabel entlang einer Z-Richtung und/oder einer Patientenzugangsrichtung zu positionieren. Die Antriebseinheit kann insbesondere dazu ausgebildet sein, den zumindest einen Teil des Patientensitzes und eines anwendungsgemäß auf dem Patientensitz positionierten Patienten entlang der Patientenzugangsrichtung in den Bildaufnahmebereich des Magnetresonanzgeräts zu transportieren. Die Raumrichtung kann jedoch auch zu der Patientenzugangsrichtung geneigt oder unter einem Winkel zu der Patientenzugangsrichtung ausgerichtet sein.

Weiterhin kann die Antriebseinheit dazu ausgebildet sein, den Patientensitz variabel entlang einer zweiten und/oder einer dritten Richtung, welche orthogonal zu der Raumrichtung ausgerichtet sind, zu transportieren. Vorzugsweise ist die Antriebseinheit dazu ausgebildet, den Patientensitz variabel entlang der Raumrichtung, der zweiten Raumrichtung und/oder der dritten Raumrichtung zu bewegen.

Vorzugsweise ist die Längsachse des Patientenaufnahmebereichs des Magnetresonanzgeräts gegenüber einer gedachten Horizontalen geneigt und mit der Raumrichtung, entlang welcher die Antriebseinheit den Patientensitz transportieren kann, derart abgestimmt, dass eine Reduktion eines Platzbedarfs gegenüber konventionellen Magnetresonanzvorrichtung, bei welcher der Patient mittels einer Patientenlagerungsvorrichtung entlang einer Horizontalen transportiert wird, ermöglicht wird.

Das erfindungsgemäße Magnetresonanzgerät teilt die Vorteile des erfindungsgemäßen Patientensitzes.

Insbesondere können das erfindungsgemäße Magnetresonanzgerät und der erfindungsgemäße Patientensitz auf vorteilhafte Weise eine Reduktion eines für eine Magnetresonanzuntersuchung erforderlichen Platzbedarfs bereitstellen und einen mit der Patientenpositionierung assoziierten Arbeitsaufwand reduzieren.

In einer Ausführungsform weist das erfindungsgemäße Magnetresonanzgerät ein Sicherungselement auf, welches dazu ausgebildet ist, ein Ausmaß einer relativen Ausrichtung des ersten Abschnitts zu dem zweiten Abschnitt zu begrenzen, um eine Kollision des Patientensitzes und/oder eines anwendungsgemäß auf dem Patientensitz positionierten Patienten mit einem Gehäuseabschnitt des Magnetresonanzgeräts zu vermeiden.

Das Sicherungselement kann beispielsweise einen Stift, einen Bolzen, einen Rastmechanismus, eine Schraube, ein Anschlagselement, ein Dämpfungselement oder dergleichen umfassen. Das Sicherungselement kann dazu ausgebildet sein, mit dem Verbindungselement und/oder der Positionierungseinheit des Patientensitzes zu interagieren oder mechanisch einzugreifen. Es ist ebenso vorstellbar, dass das Sicherungselement ein Teil des Verbindungselements und/oder der Positionierungseinheit bildet.

In einer Ausführungsform ist das Sicherungselement dazu ausgebildet, eine Bewegung des Verbindungselements und/oder der Positionierungseinheit zu begrenzen. Das Sicherungselement kann dazu ausgebildet sein, mechanisch mit dem Verbindungselement und/oder der Positionierungseinheit einzugreifen. Das Sicherungselement kann dazu ausgebildet sein, eine Bewegungsspielraum zwischen dem ersten Abschnitt und dem zweiten Abschnitt permanent zu begrenzen. Es ist jedoch ebenso vorstellbar, dass das Sicherungselement dazu ausgebildet ist, in Abhängigkeit eines Steuerbefehls in eine Sicherungsposition überführt zu werden, in der das Sicherungselement die relative Ausrichtung oder Bewegung des ersten Abschnitts zu dem zweiten Abschnitt begrenzt.

Das Sicherungselement kann einen geeigneten Antrieb aufweisen, welcher mittels einer Steuereinheit des Magnetresonanzgeräts und/oder des Patientensitzes ansteuerbar ist. Der Antrieb des Sicherungselements kann dazu ausgebildet sein, das Sicherungselement zu aktiveren oder das Ausmaß der relativen Ausrichtung des ersten Abschnitts zu dem zweiten Abschnitt mittels Aktivierung des Sicherungselements zu begrenzen. Insbesondere kann der Antrieb eine Signalverbindung mit einer eigenständigen oder einer in das Magnetresonanzgerät integrierten Steuereinheit aufweisen. Der Antrieb kann beispielsweise eine mechanische Feder umfassen und/oder elektrisch, mechanisch, pneumatisch oder hydraulisch ansteuerbar ausgestaltet sein.

Durch das Bereitstellen eines Sicherungselements lässt sich eine Kollision des Patientensitzes und/oder eines anwendungsgemäß auf dem Patientensitz positionierten Patienten mit einem Gehäuseabschnitt des Magnetresonanzgeräts bei dem Transport des Patientensitzes in Richtung des Patientenaufnahmebereichs auf vorteilhafte Weise vermeiden. Dadurch kann eine eigenständige Positionierung des Patienten auf dem Patientensitz ermöglicht werden, ohne dass weitere Arbeitsschritte zur Kontrolle der Sicherheit durch das medizinische Personal erforderlich sind.

In einer weiteren Ausführungsform des erfindungsgemäßen Magnetresonanzgeräts sind die Antriebseinheit und die Positionierungseinheit dazu ausgebildet, den Patientensitz entlang der Raumrichtung dem Patientenaufnahmebereich des Magnetresonanzgeräts zuzuführen und gleichzeitig den ersten Abschnitt variabel relativ zu dem zweiten Abschnitt zu bewegen.

Vorzugsweise sind die Bewegung des Patientensitzes mittels der Antriebseinheit und die relative Positionierung des ersten Abschnitts und des zweiten Abschnitts mittels der Positionierungseinheit zeitlich miteinander abgestimmt. Insbesondere können die Bewegung des Patientensitzes mittels der Antriebseinheit und die relative Positionierung des ersten Abschnitts und des zweiten Abschnitts mittels der Positionierungseinheit zeitlich überlappen oder sich überschneiden.

In einer Ausführungsform weist das Magnetresonanzgerät eine Steuereinheit auf, welche dazu ausgebildet ist, die Antriebseinheit und die Positionierungseinheit anzusteuern, den Patientensitz entlang der Raumrichtung auf einen Patientenaufnahmebereich des Magnetresonanzgeräts zuzuführen und gleichzeitig den ersten Abschnitt relativ zu dem zweiten Abschnitt zu positionieren. Die Steuereinheit kann als eine eigenständige Steuereinheit ausgestaltet sein oder in eine Steuereinheit des Magnetresonanzgeräts integriert sein.

Vorzugsweise ist die Steuereinheit dazu ausgebildet, eine Kollision des Patientensitzes und/oder eines anwendungsgemäß auf dem Patientensitz positionierten Patienten mit einem Gehäuseabschnitt des Magnetresonanzgeräts zu vermeiden.

In einer Ausführungsform sind die Antriebseinheit und die Positionierungseinheit dazu ausgebildet, einen Abschnitt des Patientensitzes mit einer Kopfstütze und/oder dem Kopf eines anwendungsgemäß auf dem Patientensitz positionierten Patienten in einem Bildgebungsvolumen des Magnetresonanzgeräts anzuordnen.

In einer weiteren Ausführungsform sind die Antriebseinheit und/oder die Positionierungseinheit dazu ausgebildet, eine diagnostisch relevante Körperregion eines anwendungsgemäß auf dem Patientensitz positionierten Patienten zumindest entlang einer ersten Raumrichtung und einer zweiten Raumrichtung, welche orthogonal zu der ersten Raumrichtung ausgerichtet ist, automatisch in einem Bildgebungsvolumen des Magnetresonanzgeräts zu positionieren.

Mittels eines erfindungsgemäßen Magnetresonanzgeräts lässt sich eine diagnostisch relevante Körperregion eines Patienten automatisch entlang einer komplexen zweidimensionalen oder dreidimensionalen Bewegungstrajektorie in dem Bildgebungsvolumen des Magnetresonanzgeräts positionieren. Dadurch kann eine Vielzahl von aufeinanderfolgenden, eindimensionalen Bewegungen des Patientensitzes bei der Positionierung des Patienten in dem Bildaufnahmebereich vermieden und ein Zeitaufwand für die Patientenpositionierung reduziert werden.

In einer Ausführungsform umfasst das erfindungsgemäße Magnetresonanzgerät zumindest einen Sensor, welcher dazu ausgebildet ist, eine Information über eine relative Position des Patientensitzes und/oder eines anwendungsgemäß auf dem Patientensitz positionierten Patienten und einem Gehäuseabschnitt des Magnetresonanzgeräts zu ermitteln.

Der Sensor kann mittels einer Signalverbindung mit einer Steuereinheit des Magnetresonanzgeräts verbunden sein. Vorzugsweise weist die Steuereinheit eine Recheneinheit auf, welche dazu ausgebildet ist, Daten mit einer von dem Sensor erfassten Information über die relative Position des Patientensitzes und/oder des anwendungsgemäß auf dem Patientensitz positionierten Patienten und dem Gehäuseabschnitt des Magnetresonanzgeräts zu verarbeiten und in Abhängigkeit der von dem Sensor erfassten Daten einen Positionierungsanweisung zu bestimmen. Die Positionierungsanweisung kann Bewegungsinformationen, insbesondere zeitabhängige Bewegungsinformationen, umfassen, welche mittels der Steuereinheit als Steuerbefehle für die Antriebseinheit und/oder die Positionierungseinheit ausgegeben werden können. Vorzugsweise ermöglicht die Positionierungsanweisung ein Positionieren einer diagnostisch relevanten Körperregion des Patienten in dem Bildgebungsvolumen des Magnetresonanzgeräts, ohne eine Kollision des Patientensitzes und/oder des Patienten mit einem Gehäuseabschnitt des Magnetresonanzgeräts zu verursachen.

Der Sensor kann beispielsweise als ein optischer Sensor, insbesondere ein Abstandssensor oder eine Kamera, ausgestaltet sein. Vorzugsweise umfasst das Magnetresonanzgerät eine Mehrzahl von Sensoren, welche dazu ausgebildet sind, die relative Position des Patientensitzes und/oder des anwendungsgemäß auf dem Patientensitz positionierten Patienten und dem Gehäuseabschnitt des Magnetresonanzgeräts zu ermitteln. Die Recheneinheit kann dazu ausgebildet sein, ein Positionsbestimmungsverfahren und/oder ein Bilderkennungsverfahren zum Ermitteln einer relativen Position des Patientensitzes und/oder des anwendungsgemäß auf dem Patientensitz angeordneten Patienten zu einem Gehäuseabschnitt des Magnetresonanzgeräts anzuwenden, um die Positionierungsanweisung zu bestimmen.

In einer weiteren Ausführungsform ist eine Recheneinheit des Magnetresonanzgeräts dazu ausgebildet, die Positionierungsanweisung in Abhängigkeit eines Patientenmodells zu ermitteln. Die Recheneinheit kann mit einer Datenbank, insbesondere einem Radiologischen Informationssystem (RIS), einem lokalen Datenspeicher, einer Cloud oder einer vergleichbaren Datenbank verbunden sein, welche Patienteninformationen und/oder ein Patientenmodell enthält.

Insbesondere kann die Recheneinheit dazu ausgebildet sein, in Abhängigkeit der von dem Sensor erfassten Daten und/oder der Patienteninformationen ein Patientenmodell auszuwählen und/oder anzupassen. Die Recheneinheit kann weiterhin dazu ausgebildet sein, die Positionierungsanweisung in Abhängigkeit des ausgewählten und/oder angepassten Patientenmodells zu bestimmen. Die Bestimmung der Positionierungsanweisung kann weiterhin auf Basis einer Information über eine Abmessung des Patientensitzes und des Magnetresonanzgeräts erfolgen, welche mittels der Datenbank bereitgestellt werden kann. Es ist jedoch ebenso vorstellbar, dass die Bestimmung der Positionierungsanweisung auf Basis des Patientenmodells und der von dem Sensor erfassten Daten erfolgt.

Ein Gehäuseabschnitt des Magnetresonanzgeräts kann beispielsweise ein Teil einer Einhausung des Hauptmagneten oder des Patientenaufnahmebereichs darstellen.

Insbesondere kann ein Gehäuseabschnitt ein beliebiger Teil oder Abschnitt des Magnetresonanzgeräts sein, welcher in der Nähe zu einer mittels der Antriebseinheit und/oder der Positionierungseinheit realisierten Bewegungstrajektorie des Patientensitzes angeordnet ist und/oder in eine mögliche Bewegungsbahn des Patientensitzes hineinragt.

Ein erfindungsgemäßes Magnetresonanzgerät mit einem Sensor kann eine Kollision zwischen dem Patientensitz und/oder dem Patienten mit einem Gehäuseabschnitt auf vorteilhafte Weise vermeiden. Durch eine Ermittlung einer Positionierungsanweisung und eine Ausgabe entsprechender Steuerbefehle kann eine diagnostisch relevante Körperregion zeiteffizient und automatisch in dem Bildgebungsvolumen positioniert werden, wodurch eine manuelle Überwachung und/oder Durchführung der Patientenpositionierung durch das medizinische Personal auf vorteilhafte Weise entfallen kann.

In einer weiteren Ausführungsform weist das erfindungsgemäße Magnetresonanzgerät einen Sensor auf, welcher dazu ausgebildet ist, eine Information über eine relative räumliche Anordnung des zumindest einen Antennenelements zu einem Abschnitt des Patientensitzes zu bestimmen.

Der Sensor kann dazu ausgebildet sein, Daten zu erfassen, welche eine Information über die relative räumliche Anordnung des zumindest einen Antennenelements zu dem Abschnitt des Patientensitzes enthalten. Der Sensor kann mit einem Sensor einer oben beschriebenen Ausführungsform übereinstimmen. Beispielsweise kann der Sensor als ein optischer Sensor (z. B. eine Infrarot-Kamera, eine 2D Kamera, eine 3D Kamera) oder ein Abstandssensor (z. B. ein Laser-Abstandssensor, ein Positionsgeber oder ein Hall-Sensor) ausgestaltet sein. Der Sensor kann aber auch als ein beliebiger mechanischer Sensor ausgestaltet sein, welcher dazu ausgebildet ist, eine relative Bewegung des zumindest einen Antennenelements zu einem Abschnitt des Patientensitzes zu ermitteln. Beispielweise kann der mechanische Sensor dazu ausgebildet sein, infolge der relativen Bewegung des zumindest einen Antennenelements und des Abschnitts des Patientensitzes verformt zu werden. Es ist jedoch ebenso vorstellbar, dass der Sensor eine in Abhängigkeit der relativen Bewegung des zumindest einen Antennenelements und des Abschnitts des Patientensitzes veränderliche Messstrecke aufweist. Der Sensor kann weiterhin einen Messumformer aufweisen, welcher eine von dem Sensor erfasste Information über die relative räumliche Anordnung des zumindest einen Antennenelements zu dem Abschnitt des Patientensitzes in ein elektrisches Signal, vorzugsweise digitale Daten, umwandelt.

Der Abschnitt des Patientensitzes kann ein erster Abschnitt und/oder ein zweiter Abschnitt, insbesondere eine Sitzfläche, eine Lehne und/oder eine Kopfstütze, darstellen. In einer bevorzugten Ausführungsform ist der Abschnitt eine Kopfstütze des Patientensitzes.

Erfindungsgemäß sind die Antriebseinheit und/oder die Positionierungseinheit dazu ausgebildet, die diagnostisch relevante Körperregion des Patienten in Abhängigkeit der Information über die relative räumliche Anordnung des zumindest einen Antennenelements zu dem Abschnitt des Patientensitzes in dem Bildgebungsvolumen zu positionieren.

Das Magnetresonanzgerät kann eine Recheneinheit umfassen, welche dazu ausgebildet ist, erfasste Daten oder elektrische Signale des Sensors zu verarbeiten. Vorzugsweise ist die Recheneinheit dazu ausgebildet, eine Positionierungsanweisung zu ermitteln, anhand derer die diagnostisch relevante Körperregion des Patienten in dem Bildgebungsvolumen positioniert werden kann. Die Positionierungsanweisung kann entsprechende Steuerbefehle für die Antriebseinheit und/oder die Positionierungseinheit umfassen. Es ist ebenso vorstellbar, dass die Recheneinheit und/oder eine Steuereinheit dazu ausgebildet sind, die Positionierungsanweisung in entsprechende Steuerbefehle umzuwandeln. Vorzugsweise werden die Steuerbefehle mittels einer Steuereinheit an die Antriebseinheit und/oder die Positionierungseinheit ausgegeben. Die Recheneinheit und die Steuereinheit können als eine selbstständige Einheit ausgestaltet sein oder in eine Steuereinheit des Magnetresonanzgeräts integriert sein.

Ein erfindungsgemäßes Magnetresonanzgerät erlaubt eine automatische Erkennung einer diagnostisch relevanten Körperregion und/oder einer Abmessung der diagnostisch relevanten Körperregion des Patienten in Abhängigkeit der relativen Anordnung des zumindest einen Antennenelements zu dem Abschnitt des Patientensitzes. Dadurch lässt sich eine besonders kosteneffiziente und/oder technisch robuste, automatisierte Isozentrierung (d. h. eine anwendungsgemäße Anordnung der diagnostisch relevanten Körperregion in dem Bildgebungsvolumen) der diagnostisch relevanten Körperregion erreichen.

Gemäß der vorliegenden Erfindung weist das Magnetresonanzgerät einen Arretierungsmechanismus mit einem ersten Teil und einem zweiten Teil auf. Der erste Teil und der zweite Teil des Arretierungsmechanismus sind komplementär zueinander ausgestaltet und dazu ausgebildet, mechanisch ineinanderzugreifen.

Der erste Teil und der zweite Teil des Arretierungsmechanismus können mittels einer reversiblen mechanischen Verbindung, insbesondere einer kraftschlüssigen und/oder einer formschlüssigen Verbindung, miteinander verbindbar sein. Beispielsweise können der erste Teil und der zweite Teil als komplementäre Gegenstücke eines Rastmechanismus, eines Steckmechanismus, eines Klemmmechanismus, eines Einhängemechanismus oder dergleichen ausgestaltet sein.

In einer bevorzugten Ausführungsform ist der erste Teil trichterförmig ausgestaltet, während der zweite Teil kugelförmig oder kegelförmig ausgestaltet ist. Es ist vorstellbar, dass der trichterförmige erste Teil dazu ausgebildet ist, den kugelförmigen zweiten Teil aufzunehmen und dabei in Richtung eines Mittelpunkts des trichterförmigen ersten Teils zu zentrieren. Weiterhin kann der trichterförmige erste Teil an einem engsten Querschnitt eine Vertiefung, insbesondere eine Ausbuchtung oder einen zylindrischen Hohlraum, aufweisen. Die Vertiefung kann dazu ausgebildet sein, den kugelförmigen zweiten Teil aufzunehmen und eine Bewegung des zweiten Teils entlang von zwei zueinander orthogonal ausgerichteten Raumrichtungen, insbesondere der Y-Richtung und der X-Richtung des Magnetresonanzgeräts, zu verhindern. Vorzugsweise ermöglicht der trichterförmige erste Teil ein begrenztes Rotieren des kugelförmigen ersten Teils, sodass eine mit dem ersten Teil verbundene Kopfstütze und/oder ein mit dem ersten Teil verbundener zweiter Abschnitt des Patientensitzes weiterhin mittels des Verbindungselements und/der der Positionierungseinheit bewegt werden können.

In einer Ausführungsform weist der erste Teil ein Anschlagselement auf, welches dazu ausgebildet ist, eine Bewegung des zweiten Teils entlang der Z-Achse des Magnetresonanzgeräts oder der Längsachse des Patientenaufnahmebereichs zu verhindern. Beispielsweise kann das Anschlagselement als eine Wandung innerhalb der Vertiefung des trichterförmigen ersten Teils oder als eine konische Wandung des trichterförmigen ersten Teils ausgestaltet sein.

Erfindungsgemäß ist der zweite Teil mechanisch mit dem Patientensitz verbunden. Beispielsweise kann der zweite Teil an dem ersten Abschnitt, dem zweiten Abschnitt und/oder der Kopfstütze befestigt sein. In einer bevorzugten Ausführungsform ist der zweite Teil an der Kopfstütze des Patientensitzes befestigt.

Der erste Teil ist innerhalb des Patientenaufnahmebereichs angeordnet und mit einem Gehäuseabschnitt des Magnetresonanzgeräts mechanisch verbunden. Vorzugsweise ist der erste Teil mechanisch mit einem Gehäuseabschnitt des Patientenaufnahmebereichs, insbesondere einer Haltestruktur für den Patientenaufnahmebereich und/oder der Magnethaltestruktur, verbunden.

Der Arretierungsmechanismus ist dazu ausgebildet, eine Bewegung eines Abschnitts des Patientensitzes entlang einer Raumrichtung zu begrenzen oder zu verhindern. Vorzugsweise ist der zweite Teil an einer Kopfstütze des Patientensitzes befestigt, sodass der Arretierungsmechanismus dazu ausgebildet ist, eine Bewegung der Kopfstütze in zumindest einer Raumrichtung, insbesondere einer Z-Richtung des Magnetresonanzgeräts oder einer Patientenzugangsrichtung, zu begrenzen.

Es ist vorstellbar, dass der Arretierungsmechanismus dazu ausgebildet ist, eine Bewegung des Abschnitts des Patientensitzes in mindestens zwei orthogonal zueinander ausgerichteten Raumrichtungen, insbesondere einer Z-Richtung und einer Y-Richtung des Magnetresonanzgeräts, zu begrenzen oder zu verhindern.

Durch einen erfindungsgemäßen Arretierungsmechanismus lässt sich der Patientensitz in dem Patientenaufnahmebereich fixieren. Somit kann eine relative Bewegung eines Abschnitts des Patientensitzes relativ zu dem Bildgebungsvolumen des Magnetresonanzgeräts während einer Magnetresonanzuntersuchung auf vorteilhafte Weise vermieden werden. Dies kann insbesondere für Patientensitze relevant sein, bei welchen eine relative Anordnung der Abschnitte durch eine Änderung einer Körperhaltung des Patienten (wie z. B. ein Zurücklehnen oder ein Vorbeugen in eine aufrechte Sitzposition) manuell durch den Patienten veränderbar ist.

Ein erfindungsgemäßer Arretierungsmechanismus erlaubt weiterhin eine Verwendung von unbefestigten Patientensitzen oder Patientensitzen mit einer leichteren und/oder weniger stabilen Konstruktion. Durch die Verwendung solcher Patientensitze lassen sich Kosten des Patientensitzes und/oder des Magnetresonanzgeräts auf vorteilhafte Weise reduzieren.

Weiterhin kann der Arretierungsmechanismus dazu ausgebildet sein, ein Schwingen und/oder Vibrieren des Patientensitzes, insbesondere der Kopfstütze des Patientensitzes, bei einem anwendungsgemäßen Eingreifen des ersten Teils mit dem zweiten Teil zu reduzieren oder zu vermeiden. Ein Schwingen und/oder Vibrieren des Patientensitzes kann beispielsweise eine Folge von elektromagnetischen und/oder mechanischen Kräften beruhend auf einer Gradienten-Aktivität des Magnetresonanzgeräts während der Magnetresonanzuntersuchung und/oder einer Bewegung des Patienten auf dem Patientensitz darstellen.

Ein erfindungsgemäßer Arretierungsmechanismus kann auf vorteilhafte Weise zu einer Stabilisierung eines Patientensitzes, insbesondere einer Kopfstütze des Patientensitzes, gegen Schwingungen und/oder Vibrationen während einer Magnetresonanzuntersuchung beitragen oder diese teilweise oder vollständig unterbinden.

In einer weiteren Ausführungsform weist der Arretierungsmechanismus des erfindungsgemäßen Magnetresonanzgeräts einen Antrieb auf, welcher dazu ausgebildet ist, in Abhängigkeit eines Aktivierungssignals ein Ineinandergreifen des ersten Teils und des zweiten Teils zu bewirken, wenn sich der Patientensitz in einer anwendungsgemäßen Position relativ zu dem Patientenaufnahmebereich zur Durchführung der Magnetresonanzmessung befindet.

Der Arretierungsmechanismus kann beispielsweise einen elektrischen, einen hydraulischen oder einen pneumatischen Antrieb aufweisen. Vorzugsweise ist der Antrieb dazu ausgebildet, den ersten Teil und den zweiten Teil zusammenzuführen, sodass der erste Teil und der zweite Teil mechanisch ineinander eingreifen oder mechanisch miteinander verbunden sind. Vorzugsweise ist der Antrieb dazu ausgebildet, den Arretierungsmechanismus von außerhalb eines von dem Hauptmagneten umschlossenen Volumens zu aktivieren. Der Antrieb kann hierfür außerhalb des Patientenaufnahmebereichs oder außerhalb eines Hauptmagnetfelds des Magnetresonanzgeräts positioniert sein. Vorzugsweise weist der Antrieb eine mechanische, eine hydraulische oder eine pneumatische Positionierungseinheit auf, welche dazu ausgebildet ist, den ersten Teil und den zweiten Teil des Arretierungsmechanismus infolge einer Aktivierung durch den Antrieb zusammenzuführen.

In einer bevorzugten Ausführungsform ist der Antrieb dazu ausgebildet, mittels eines Aktivierungssignals einer Steuereinheit des Magnetresonanzgeräts angesteuert zu werden. Die Steuereinheit kann insbesondere dazu ausgebildet sein, den Arretierungsmechanismus zu aktivieren, nachdem eine Isozentrierung der diagnostisch relevanten Körperregion des Patienten stattgefunden hat.

In einem Beispiel weist der trichterförmige erste Teil eine Positionierungseinheit auf, welche dazu ausgebildet ist, infolge einer Ansteuerung mittels des Antriebs den ersten Teil relativ zu dem zweiten Teil in Richtung des zweiten Teils zu bewegen. In einem weiteren Beispiel weist der kugelförmige zweite Teil eine Positionierungseinheit auf, welche dazu ausgebildet ist, infolge einer Ansteuerung mittels des Antriebs den zweiten Teil relativ zu dem ersten Teil in Richtung des ersten Teils zu bewegen. Beispielsweise kann die Positionierungseinheit eine Welle, insbesondere eine Spindel, umfassen, welche mittels des Antriebs entlang einer Längsrichtung des Patientenaufnahmebereichs oder einer Z-Richtung des Magnetresonanzgeräts auslenkbar ist.

Durch einen erfindungsgemäßen Arretierungsmechanismus kann auf einen manuellen Arbeitsschritt des Arretierens des Patientensitzes verzichtet werden. Weiterhin ermöglicht der erfindungsgemäße Arretierungsmechanismus einen größeren Freiraum bei der Patientenpositionierung, da die Positionierungseinheit einen Abstand zwischen dem ersten Teil und dem zweiten Teil des Arretierungsmechanismus überbrücken kann und der Patientensitz somit nicht soweit in den Patientenaufnahmebereich transportiert werden muss, bis das zweite Element an einem Anschlagselement des ersten Elements anliegt.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: ein konventionelles Magnetresonanzgerät,
- Fig. 2: eine Ausführungsform eines erfindungsgemäßen Magnetresonanzgeräts,
- Fig. 3: eine Ausführungsform eines erfindungsgemäßen Magnetresonanzgeräts,
- Fig. 4: eine Ausführungsform eines erfindungsgemäßen Patientensitzes,
- Fig. 5: eine Ausführungsform eines erfindungsgemäßen Patientensitzes,
- Fig. 6: eine Ausführungsform eines erfindungsgemäßen Patientensitzes,
- Fig. 7: eine Ausführungsform eines erfindungsgemäßen Patientensitzes,
- Fig. 8: eine Ausführungsform eines erfindungsgemäßen Magnetresonanzgeräts.

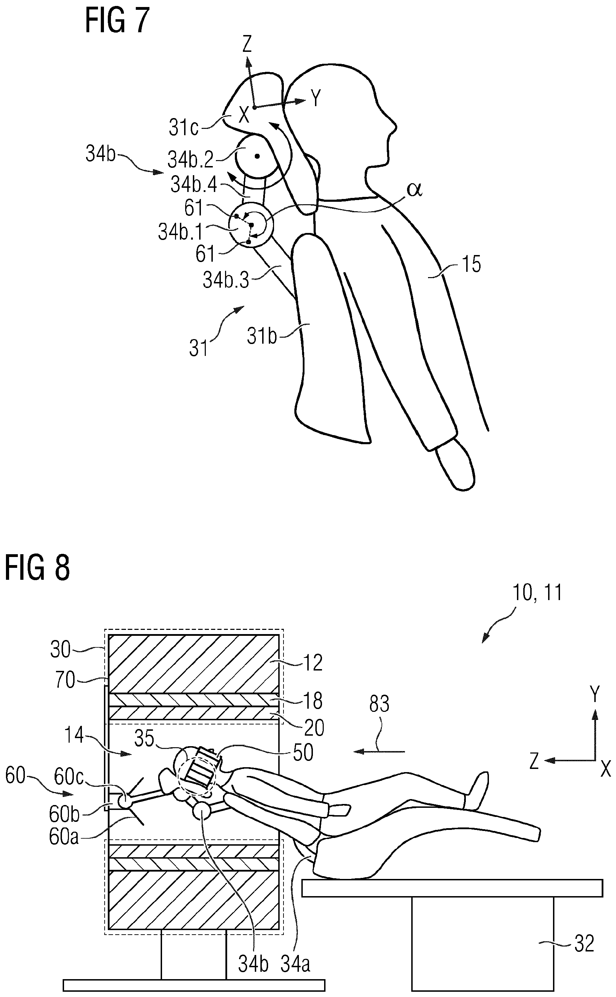

Die vorliegende Erfindung beruht auf der in Figur 8 gezeigeten Ausführungsform, die den erfindungsgemäßen Arretierungsmechanismus aufweist. Die Ausführungsformen, die in den anderen Figuren gezeigt werden, sind in ihrer Allgemeinheit nicht Bestandteil der vorliegenden Erfindung.

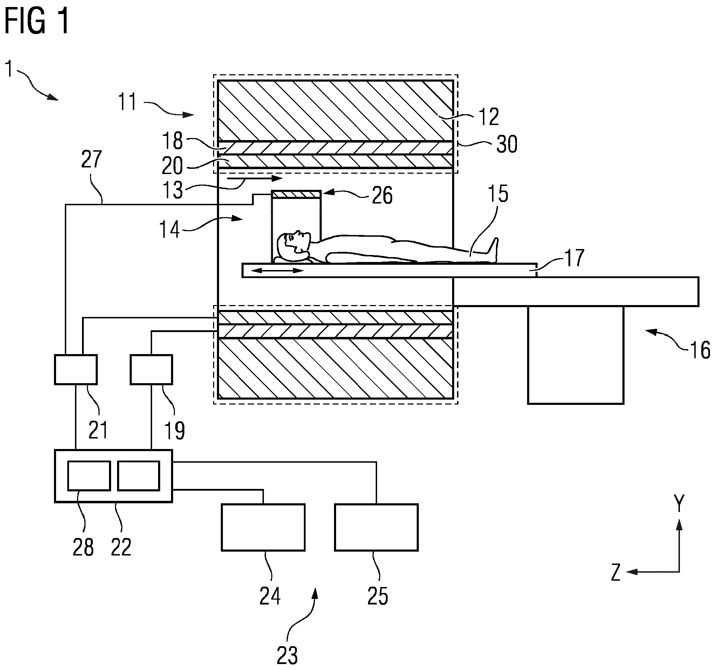

In Fig. 1 ist ein konventionelles Magnetresonanzgerät 1 dargestellt. Das Magnetresonanzgerät 1 umfasst eine Felderzeugungseinheit 11, welche einen Hauptmagneten 12 mit einem oder mehreren Permanentmagneten, Elektromagneten oder supraleitenden Magneten zur Erzeugung eines starken und insbesondere homogenen Hauptmagnetfelds 13 (B0-Magnetfeld) aufweist. Zudem umfasst das Magnetresonanzgerät 1 einen Patientenaufnahmebereich 14 zu einer Aufnahme eines Patienten 15. Der Patientenaufnahmebereich 14 ist in dem gezeigten Ausführungsbeispiel zylinderförmig ausgebildet und in einer Umfangsrichtung von dem Hauptmagneten 12 umschlossen. Grundsätzlich sind jedoch auch von diesem Beispiel abweichende Ausbildungen des Patientenaufnahmebereichs 14 vorstellbar. Der Patientenaufnahmebereich 14 kann im Wesentlichen mit einem Bildaufnahmebereich des Magnetresonanzgeräts 1 übereinstimmen.

In dem in Fig. 1 gezeigten Beispiel ist der Patient 15 mittels einer

Patientenlagerungsvorrichtung 16 des Magnetresonanzgeräts 1 in dem Patientenaufnahmebereich 14 positionierbar. Die Patientenlagerungsvorrichtung 16 weist hierfür einen horizontal bewegbaren Patiententisch 17 auf.

Die Felderzeugungseinheit 11 weist ein Gradientensystem mit wenigstens einer Gradientenspule 18 zum Erzeugen eines magnetischen Gradientenfelds auf, welches für eine Ortskodierung während einer Magnetresonanzuntersuchung verwendet wird. Die Gradientenspule 18 wird mittels einer Gradientensteuereinheit 19 des Magnetresonanzgeräts 1 angesteuert. Es ist vorstellbar, dass das Gradientensystem mehrere Gradientenspulen 18 zur Erzeugung von magnetischen Gradientenfeldern entlang unterschiedlicher, vorzugsweise orthogonal zueinander ausgerichteter, Raumrichtungen umfasst.

Die Felderzeugungseinheit 11 umfasst außerdem ein Hochfrequenzsystem mit einer Hochfrequenzspule, welche im vorliegenden Ausführungsbeispiel als fest in das Magnetresonanzgerät 1 integrierte Körperspule 20 ausgebildet ist. Die Körperspule 20 ist zu einer Anregung von Kernspins ausgelegt, die sich in dem von dem Hauptmagneten 12 erzeugten Hauptmagnetfeld 13 befinden. Die Körperspule 20 wird von einer Hochfrequenzsteuereinheit 21 des Magnetresonanzgeräts 1 angesteuert und strahlt hochfrequente Anregungspulse in den Bildaufnahmebereich, der im Wesentlichen von dem Patientenaufnahmebereich 14 des Magnetresonanzgeräts 1 gebildet ist, ein. Die Körperspule 20 kann weiterhin zum Empfang von Magnetresonanzsignalen ausgebildet sein und eine Empfangseinheit oder einen Teil einer Empfangseinheit des Magnetresonanzgeräts 1 bilden.

Zu einer Steuerung des Magnetresonanzgeräts 1, insbesondere der Gradientensteuereinheit 19 und der Hochfrequenzsteuereinheit 21, weist das Magnetresonanzgerät 1 eine Steuereinheit 22 auf. Die Steuereinheit 22 ist insbesondere dazu ausgebildet, eine Durchführung einer Bildgebungssequenz, wie z. B. einer GRE (gradient echo) Sequenz, einer TSE (turbo spin echo) Sequenz oder einer UTE (ultra-short echo time) Sequenz, zu koordinieren. Zudem umfasst die Steuereinheit 22 eine Recheneinheit 28 zu einer Auswertung von Magnetresonanzsignalen, die während einer Magnetresonanzuntersuchung mit einer Bildgebungssequenz erfasst werden.

Das Magnetresonanzgerät 1 kann eine Benutzerschnittstelle 23 umfassen, welche eine Signalverbindung mit der Steuereinheit 22 aufweist. Steuerinformationen, wie z. B. Bildgebungsparameter der Magnetresonanzuntersuchung, können auf einer Anzeigeeinheit 24, beispielsweise auf zumindest einem Monitor, der Benutzerschnittstelle 23 angezeigt werden. Die Anzeigeeinheit 24 kann insbesondere dazu ausgelegt sein, eine grafische Benutzeroberfläche mit der Darstellung einer relevanten Körperregion des Patienten 15 bereitzustellen. Weiterhin weist die Benutzerschnittstelle 23 eine Eingabeeinheit 25 auf, mittels welcher Parameter einer Magnetresonanzmessung von einem Nutzer eingegeben oder verändert werden können.

Das Magnetresonanzgerät 1 kann weitere Komponenten, wie z. B. eine Lokalspule 26, aufweisen. Die Lokalspule 26 kann in einer anwendungsgemäßen Position an einer diagnostisch oder therapeutisch relevanten Körperregion des Patienten 15 positioniert sein. Die Lokalspule 26 weist vorzugsweise eine Mehrzahl von Antennenelementen auf, welche dazu ausgebildet sind, Magnetresonanzsignale der relevanten Körperregion des Patienten 15 zu erfassen und an die Recheneinheit 28 und/oder die Steuereinheit 22 zu übermitteln. Die Lokalspule kann hierfür mittels einer elektrischen Anschlussleitung 27 oder einer anderen Signalverbindung mit der Hochfrequenzsteuereinheit 21 und der Steuereinheit 22 verbunden sein. Analog zu der Körperspule 20 kann auch die Lokalspule 26 zu einem Anregen von Kernspins in der Kieferregion 31 des Patienten 15 ausgebildet sein. Die Lokalspule 26 kann hierfür von der Hochfrequenzsteuereinheit 21 angesteuert werden.

Üblicherweise sind die Felderzeugungseinheit 11 sowie eine Magnethaltestruktur von einem Gehäuse 30 umschlossen. Das Gehäuse 30 kann dazu ausgebildet sein, Komponenten des Magnetresonanzgeräts 1 vor äußeren Einflüssen zu schützen und/oder einen Berührschutz für einen Patienten 15 bereitzustellen.

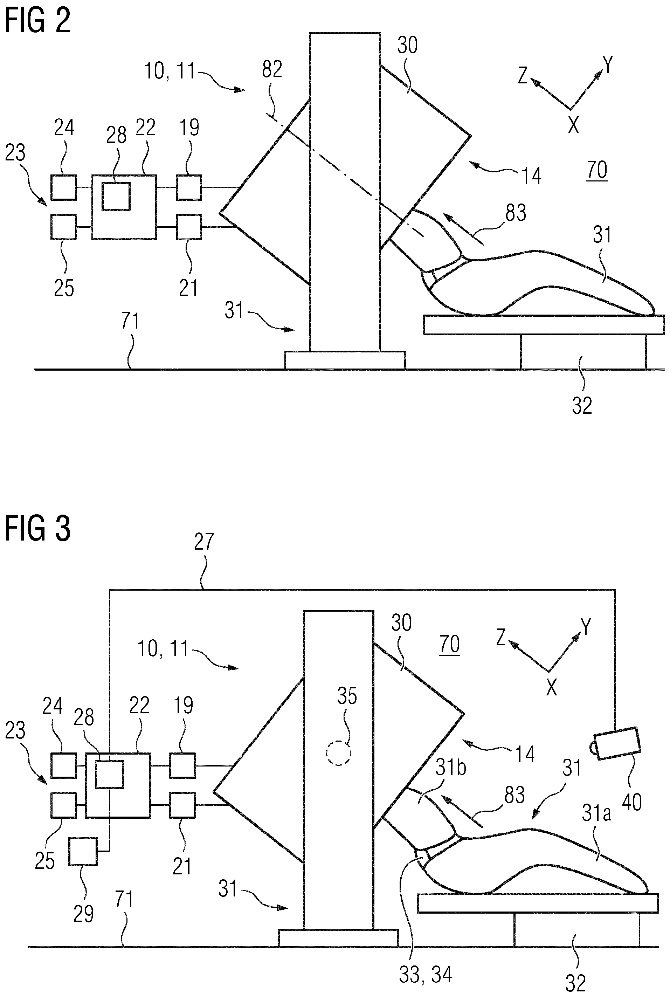

Die Fig. 2 zeigt eine Ausführungsform eines erfindungsgemäßen Magnetresonanzgeräts 10. Grundsätzlich stimmen die Funktionen und Komponenten des in Fig. 2 dargestellten Magnetresonanzgeräts 10 mit den oben beschriebenen Funktionen und Komponenten eines konventionellen Magnetresonanzgeräts 1 (siehe Fig. 1) überein.

Beispielsweise kann das Magnetresonanzgerät 10 dazu ausgebildet sein, eine Magnetresonanzuntersuchung einer Kopfregion, einer Kieferregion und/oder einer Zahnregion eines Patienten 15 durchzuführen. Das erfindungsgemäße Magnetresonanzgerät 10 kann jedoch ebenso dazu ausgebildet sein, eine kardiale Bildgebung, eine neurologische Bildgebung, eine urologische Bildgebung, eine orthopädische Bildgebung, eine Prostata-Bildgebung oder eine Bildgebung anderer Körperregionen des Patienten 15, insbesondere Extremitäten, durchzuführen.

In der in Fig. 2 gezeigten Ausführungsform ist eine Längsachse 82 des Patientenaufnahmebereichs 14 (bzw. eine Längsachse 82 des Magnetresonanzgeräts 10) gegenüber einer Horizontalen 71, insbesondere einer horizontal ausgerichteten Bodenfläche 71 eines Untersuchungsraums 70, geneigt angeordnet. Die Patientenzugangsrichtung 83 stimmt in dem vorliegenden Beispiel mit der Z-Richtung des Magnetresonanzgeräts 10 überein und weist ebenfalls eine Neigung gegenüber der Bodenfläche 71 auf. Die Neigung des Magnetresonanzgeräts 10 ermöglicht eine Magnetresonanzuntersuchung eines sitzend angeordneten Patienten und somit eine Reduktion eines Platzbedarfs des Magnetresonanzgeräts 10 mit dem Patientensitz 31.

Der Patientensitz 31 weist vorliegend eine Antriebseinheit 32 auf, welche dazu ausgebildet ist, den Patientensitz 31 variabel entlang einer Raumrichtung zu bewegen. In dem gezeigten Beispiel ist die Antriebseinheit dazu ausgebildet, den Patientensitz 31 parallel zu der Bodenfläche 71 zu bewegen. Es ist jedoch ebenso vorstellbar, dass die Antriebseinheit dazu ausgebildet ist, den Patientensitz 31 unter einem Winkel zu der Bodenfläche 71, insbesondere entlang der Z-Richtung des Magnetresonanzgeräts 10, zu bewegen. Die Antriebseinheit kann auch dazu ausgebildet sein, den Patientensitz 31 variabel entlang mehrerer Raumrichtungen, insbesondere orthogonal zueinander ausgerichteter Raumrichtungen, zu bewegen.

Die Fig. 3 zeigt eine Ausführungsform des erfindungsgemäßen Magnetresonanzgeräts 10 mit einem Sensor 40. Der Sensor 40 ist vorliegend als eine Kamera ausgestaltet, welche dazu ausgebildet ist, Bilddaten in dem Untersuchungsraum 70 mit dem Magnetresonanzgerät 10 zu erfassen und mittels der Signalverbindung 27 an die Recheneinheit 28 des Magnetresonanzgeräts 10 zu übermitteln.

Die Recheneinheit 28 ist dazu ausgebildet, eine relative Position des Patientensitzes 31 und/oder eine relative Position eines anwendungsgemäß auf dem Patientensitz positionierten Patienten und einem Gehäuseabschnitt des Gehäuses 30 des Magnetresonanzgeräts 10 in Abhängigkeit der von dem Sensor 40 erfassten Bilddaten zu ermitteln. Die Recheneinheit 28 ist weiterhin dazu ausgebildet, eine Positionierungsanweisung in Abhängigkeit der Bilddaten des Sensors 40 zu bestimmen. Die Positionierungsanweisung umfasst zeitabhängige Bewegungsinformationen, welche mittels der Steuereinheit 22 als Steuerbefehle an die Antriebseinheit 32 und/oder die Positionierungseinheit 33 des Patientensitzes 31 ausgegeben werden. Die Steuereinheit 22 kann entsprechend dazu ausgebildet sein, eine diagnostisch relevante Körperregion des Patienten mittels der Antriebseinheit 32 und/oder der Positionierungseinheit 33 in Abhängigkeit der Positionierungsanweisung in einem Bildgebungsvolumen des Magnetresonanzgeräts 10 zu positionieren. In einer bevorzugten Ausführungsform ist die Steuereinheit 22 dazu ausgebildet, den Patientensitz 31 mittels Ansteuerung der Antriebseinheit 32 und der Positionierungseinheit 33 entlang der Patientenzugangsrichtung 83 dem Patientenaufnahmebereich 14 des Magnetresonanzgeräts 10 zuzuführen und gleichzeitig den zweiten Abschnitt 31b relativ zu dem ersten Abschnitt 31a zu positionieren.

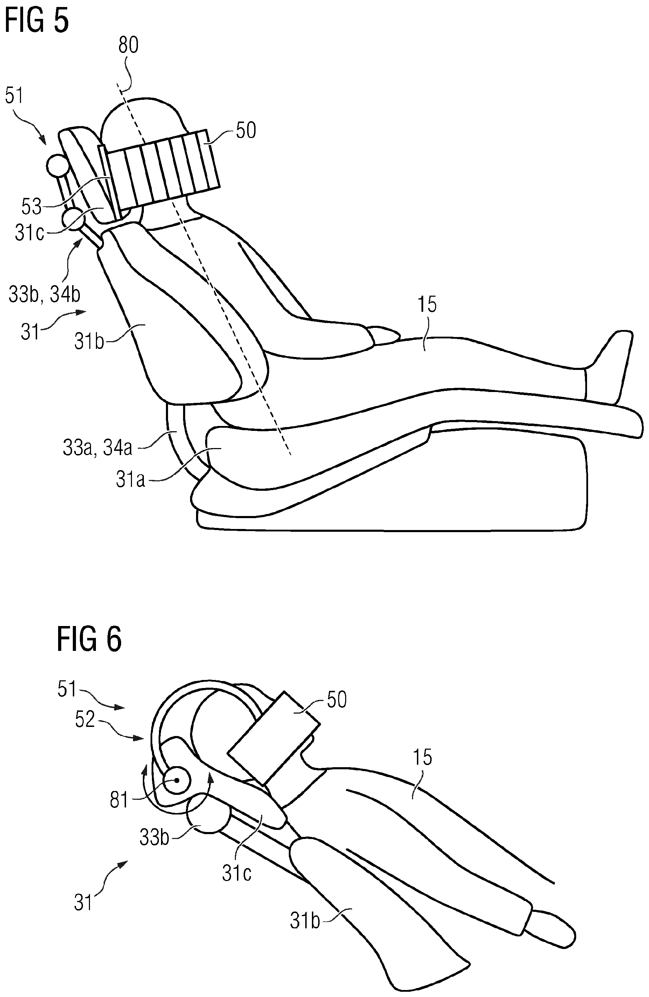

Es ist ebenso vorstellbar, dass der Sensor 40 dazu ausgebildet ist, eine Information über eine relative räumliche Anordnung eines Antennenelements 50 (siehe Fig. 5) zu einem Abschnitt des Patientensitzes 31 zu erfassen. Im Falle einer Kamera als Sensor 40 kann die Recheneinheit 28 dazu ausgebildet sein, die relative räumliche Anordnung des Antennenelements 50 zu einem Abschnitt des Patientensitzes 31 in Abhängigkeit der von dem Sensor 40 erfassten Bilddaten zu bestimmen. Die Recheneinheit 28 kann ferner dazu ausgebildet sein, eine Positionierungsanweisung zu ermitteln, anhand derer die diagnostisch relevante Körperregion des Patienten in dem Bildgebungsvolumen 35 des Magnetresonanzgeräts 10 positioniert werden kann. Wie zuvor beschrieben kann die Steuereinheit 22 dazu ausgebildet sein, entsprechende Steuerbefehle in Abhängigkeit der Positionierungsanweisung an die Antriebseinheit 32 und/oder die Positionierungseinheit 33 auszugeben.

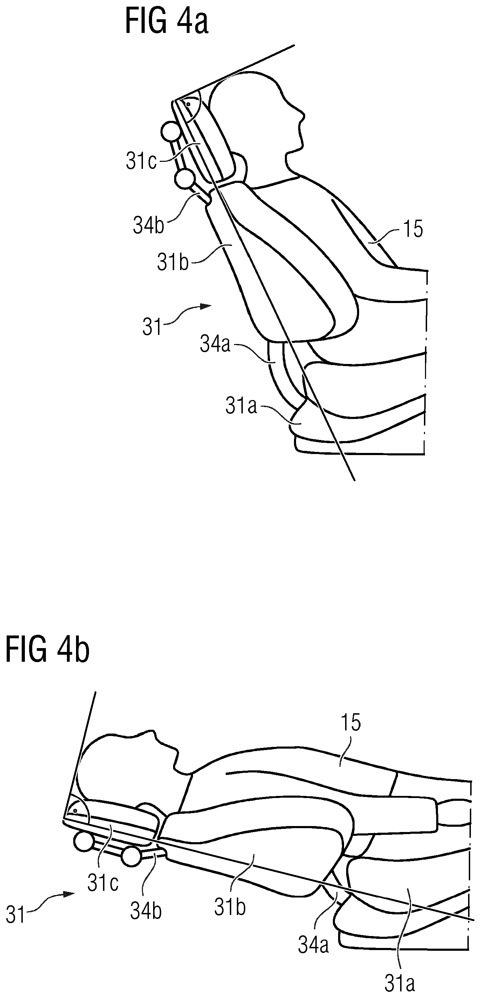

Die Fig. 4a und die Fig. 4b zeigen eine Ausführungsform des erfindungsgemäßen Patientensitzes 31 mit einer Sitzfläche 31a, einer Lehne 31b und einer Kopfstütze 31c. Die Sitzfläche 31a und die Lehne 31b sind mittels eines Verbindungselements 34a verbunden, während die Lehne 31b und die Kopfstütze 31c mittels eines Verbindungselements 34b verbunden sind. Ein Patient 15 ist anwendungsgemäß auf dem Patientensitz 31 positioniert.

Die Verbindungselemente 34a und 34b können dazu ausgebildet sein, eine variable relative Bewegung zwischen den Abschnitten 31a, 31b und 31c des Patientensitzes 31 zu ermöglichen. In dem gezeigten Beispiel ist das Verbindungselement 34a dazu ausgebildet, eine Bewegung der Lehne 31b relativ zu der Sitzfläche 31a zu ermöglichen. Das Verbindungselement 34b ermöglicht hingegen eine Bewegung der Kopfstütze 31c relativ zu der Lehne 31b. Vorliegend umfasst das Verbindungselement 34a ein Gelenk, welches ein Verkippen der Lehne 31b gegenüber der Sitzfläche 31a entlang einer vorbestimmten Bewegungstrajektorie ermöglicht. Das Verbindungselement 34b umfasst eine Mehrzahl von Gelenken, welche ein Positionieren und Verkippen der Kopfstütze 31c gegenüber der Lehne 31b ermöglichen.

In dem in Fig. 4a gezeigten Beispiel befindet sich der Patient 15 im Wesentlichen in einer aufrechten Sitzposition. Es ist vorstellbar, dass die Längsachse 82 des Patientenaufnahmebereichs 14 derart geneigt ist, dass eine Magnetresonanzuntersuchung des Patienten 15 in der aufrechten Sitzposition durchgeführt werden kann. Vorzugsweise ist der Patientensitz 31 dazu ausgebildet, eine relative Anordnung des Kopfes des Patienten 15 zu der Kopfstütze 31c bei einem wie in Fig. 4b gezeigten Zurücklehnen des Patienten 15 auf dem Patientensitz 31 zu vermeiden. Dadurch können Schritte der Vorbereitung des Patienten 15, wie z. B. eine Anordnung von Lokalspulen 26 oder Antennenelementen 50, bereits an einem aufrecht sitzenden Patienten 15 erfolgen, wodurch ein Aufwand für medizinisches Personal reduziert werden kann.

In einer bevorzugten Ausführungsform sind die Verbindungselemente 34a und 34b dazu ausgebildet ist, die Kopfstütze 31c und die Lehne 31b derart variabel relativ zu der Sitzfläche 31a zu bewegen, dass eine relative Position des Kopfes des Patienten 15 zu der Kopfstütze 31c beim Zurücklehnen des Patienten 15 im Wesentlichen unverändert bleibt. Wie in der Fig. 4a und der Fig. 4b gezeigt, bleibt ein Winkel zwischen der Kopfstütze 31 und einer Tangente an den höchsten Punkt des Kopfes des Patienten 15 im Wesentlichen unverändert, wenn sich der Patient 15 auf dem Patientensitz 31 zurücklehnt.

In allen hierin beschriebenen Ausführungsformen kann der erfindungsgemäße Patientensitz 31 passive oder rein mechanische Verbindungselemente 34 aufweisen, welche eine relative Bewegung zwischen den Abschnitten 31a, 31b und/oder 31c (31a-c) durch Interaktion eines Patienten 15 oder eines Mitglieds des medizinischen Personals ermöglichen. Ebenso können die Verbindungselemente 34 mit aktiven Positionierungseinheiten 33 gekoppelt sein, welche einen Antrieb aufweisen und eine automatisierte relative Bewegung der Abschnitte 31a, 31b und/oder 31c erlauben.

Die Fig. 5 zeigt eine Ausführungsform des erfindungsgemäßen Patientensitzes 31 mit einem Führungselement 53, welches dazu ausgebildet ist, das Antennenelement 50 der Hochfrequenzeinheit 51 variabel relativ zu der Kopfstütze 31c zu bewegen.

Das Führungselement 53 und das Antennenelement 50 sind in diesem Beispiel mechanisch mit der Kopfstütze 31c des Patientensitzes 31 verbunden. Das Führungselement 53 ist als ein Scharnier ausgeführt, welches ein Schwenken oder Rotieren des Antennenelements 50 um eine durch das Führungselement 53 definierte Achse (z. B. eine parallel zu einer Sagittalebene und/oder einer Frontalebene des Oberkörpers des Patienten 15 ausgerichtete Achse) ermöglicht. Das Antennenelement 50 lässt sich somit von der Seite an den Kopf des Patienten 15 anlegen oder sich in einem vorbestimmten Abstand zu dem Kopf des Patienten 15 positionieren.

In einer bevorzugten Ausführungsform weist der Patientensitz 31 zumindest zwei Antennenelemente 50 auf (nicht gezeigt), welche an gegenüberliegenden Seiten der Kopfstütze 31c angeordnet sind und den Kopf des anwendungsgemäß auf dem Patientensitz 31 positionierten Patienten 15 von gegenüberliegenden Seiten flankieren.

Es ist vorstellbar, dass das Führungselement 53 und/oder die Kopfstütze 31c ein Lager aufweisen, welches dazu ausgebildet ist, das Antennenelement 50 relativ entlang einer Parallelen zu der Sagittalebene und/oder der Frontalebene, insbesondere entlang einer Parallelen zu einer Schnittgeraden 80 der Sagittalebene mit der Frontalebene, des Oberkörpers des Patienten 15 zu bewegen. Dadurch kann das Antennenelement 50 neben einem Dentalbereich und/oder Kieferbereich des Patienten 15 wie in Fig. 5 gezeigt, auch an weiteren Bereichen des Kopfes des Patienten 15 angeordnet werden.

Es ist vorstellbar, dass ein Führungselement 53 mit einem Antennenelement 50 alternativ oder zusätzlich an der Lehne 31b und/oder der Sitzfläche 31a angeordnet ist, um eine Magnetresonanzuntersuchung weiterer oder anderer Körperregionen des Patienten 15 zu ermöglichen.

In der in Fig. 5 gezeigten Ausführungsform des Patientensitzes 31 lassen sich die Kopfstütze 31c, die Lehne 31b und die Sitzfläche 31a mittels der Verbindungselemente 34a und 34b durch einen Patienten 15 oder ein Mitglied des medizinischen Personals variabel relativ zueinander bewegen. Es ist jedoch ebenso vorstellbar, dass ein oder mehrere Verbindungselemente 34 als Positionierungseinheiten 33 ausgestaltet sind oder eine Positionierungseinheit 33 aufweisen. Dadurch kann eine variable relative Bewegung der Kopfstütze 31c, der Lehne 31b und/oder der Sitzfläche 31a automatisiert erfolgen. Die in Fig. 5 gezeigten Positionierungseinheiten 33a und/oder 33b können beispielsweise mittels in den Patientensitz 31 integrierter Antriebe infolge einer Ansteuerung durch die Steuereinheit 22 betätigt oder aktiviert werden.

Die Fig. 6 zeigt eine Ausführungsform, bei welcher die Hochfrequenzeinheit 51 des erfindungsgemäßen Patientensitzes 31 einen Schwenkmechanismus 52 aufweist. Der Schwenkmechanismus 52 ist dazu ausgebildet, das Antennenelement 50 um eine im Wesentlichen zu einer medialen Richtung des Patienten 15 parallel ausgerichteten Achse 81 zu schwenken oder zu rotieren. Das Antennenelement 50 kann mittels eines Führungselements, insbesondere eines Gelenks (nicht gezeigt), mit dem Schwenkmechanismus 52 verbunden sein, um ein Anpassen einer räumlichen Position und/oder Ausrichtung des Antennenelements 50 relativ zu dem Schwenkmechanismus 52 zu ermöglichen.

Vorzugsweise ist der Schwenkmechanismus 52 in die Kopfstütze 31c des Patientensitzes 31 integriert. Es ist jedoch ebenso vorstellbar, dass der Schwenkmechanismus 52 in andere Abschnitte des Patientensitzes 31 integriert ist.

Die Hochfrequenzeinheit 51 kann selbstverständlich eine Mehrzahl von Antennenelementen 50 umfassen. Vorzugsweise weist die Hochfrequenzeinheit 51 zwei Antennenelemente 50 auf, welche von dem Schwenkmechanismus 52 an gegenüberliegenden Seiten der Kopfstütze 31c gehalten werden und den Kopf des anwendungsgemäß in dem Patientensitz 31 positionierten Patienten 15 von gegenüberliegenden Seiten flankieren.

Vorzugsweise stellt die Hochfrequenzeinheit 51 des Patientensitzes 31 ein Teil des Hochfrequenzsystems des Magnetresonanzgeräts 10 dar. Beispielsweise können ein oder mehrere Antennenelemente 50 des Patientensitzes 31 mittels einer Signalverbindung mit der Hochfrequenzsteuereinheit 21 des Magnetresonanzgeräts 10 (siehe Fig.1 bis 3) verbunden sein und von dieser angesteuert werden. Es ist insbesondere vorstellbar, dass die Hochfrequenzeinheit 51 mittels einer in den Patientensitz 31 integrierten elektrischen Anschlussleitung mit der Hochfrequenzsteuereinheit 21 verbunden ist, um eine Ansteuerung der Antennenelemente 50 zu ermöglichen. Durch die Integration der Antennenelemente 50 in den Patientensitz 31 lassen sich in einem Freiraum des Patientenaufnahmebereichs 14 befindliche, elektrische Leitungen auf vorteilhafte Weise vermeiden.

Eine Hochfrequenzeinheit 51 mit einem Antennenelement 50 gemäß Fig. 5 oder Fig. 6 ermöglicht eine Anordnung des Antennenelements 50 in einer anwendungsgemäßen Position an der diagnostisch relevanten Körperregion durch den Patienten 15. Selbst wenn die Anordnung des Antennenelements 50 am Patienten 15 durch ein Mitglied des medizinischen Personals erfolgt, muss das Antennenelement 50 lediglich in die anwendungsgemäße Position geklappt oder geschwenkt werden, bevor der Patient 15 mittels des Patientensitzes 31 in die anwendungsgemäße Position zur Durchführung der Magnetresonanzuntersuchung in den Patientenaufnahmebereich 14 transportiert wird.

Die Fig. 7 zeigt eine Ausführungsform eines erfindungsgemäßen Patientensitzes 31 mit einem Verbindungselement 34b. Das Verbindungselement 34b ist vorliegenden dazu ausgebildet, die Kopfstütze 31c relativ zu der Lehne 31b des Patientensitzes zu bewegen. Das Verbindungselement 34b weist insbesondere zwei Lager oder Gelenke 34b.1 und 34b.2 auf, welche eine Positionierung und Ausrichtung der Kopfstütze 31c relativ zu der Lehne 31b ermöglichen.

Beispielsweise ist das Gelenk 34b.2 dazu ausgebildet, ein Rotieren der Kopfstütze 31c um eine durch das Gelenk 34b.2 definierte Rotationsachse zu ermöglichen. Vorzugsweise ist die durch das Gelenk 34b.2 definierte Rotationsachse im Wesentlichen parallel zu einer medialen Richtung des Patienten 15, insbesondere einer X-Richtung des Patientensitzes 31 und/oder des Magnetresonanzgeräts 10 (siehe Fig. 3), ausgerichtet. Es ist jedoch ebenso vorstellbar, dass das Gelenk 34b.2 als ein Kugelgelenk ausgestaltet ist, welches eine im Wesentlichen dreidimensional Ausrichtung der Kopfstütze 31c gegenüber der Lehne 31b ermöglicht.

Das Gelenk 34b.1 ist vorzugsweise dazu ausgebildet, eine Änderung eines Winkels zwischen zwei Streben 34b.3 und 34b.4, welche die Kopfstütze 31c mit der Lehne 31b verbinden, zu ermöglichen.

In einer bevorzugten Ausführungsform weisen die Gelenke 34b.1 und/oder 34b.2 mechanische Widerstände auf, welche eine relative Bewegung der Kopfstütze 31c zu der Lehne 31b, erst ab Überschreiten eines vorbestimmten Kraftaufwands erlauben. Dadurch kann der Patientensitz 15 abhängig von einer Körperhaltung des Patienten 15, insbesondere einer durch den Körper des Patienten 15 auf die Abschnitte 31c und 31b ausgeübten Gewichtskraft, dauerhaft oder für die Dauer der Magnetresonanzuntersuchung in einer gewünschten Konfiguration gehalten werden. Die gewünschte Konfiguration kann insbesondere dadurch charakterisiert sein, dass eine diagnostisch relevante Körperregion des Patienten 15, wie z. B. ein Abschnitt des Gehirns, ein Abschnitt einer Kieferregion und/oder ein Abschnitt eines Zahnbogens, in dem Bildgebungsvolumen 35 des Magnetresonanzgeräts 10 (siehe Fig. 3 und Fig. 8) positioniert sind. Es ist vorstellbar, dass das Verbindungselement 34b Mittel zur Einstellung der mechanischen Widerstände (nicht gezeigt) der Gelenke 34b.1 und/oder 34b.2 aufweist. Solche Mittel können beispielsweise einen Schraubmechanismus umfassen, welcher eine Anpassung einer Reibungskraft zwischen beweglichen Teilen der Gelenke 34b.1 und/oder 34b.2 ermöglicht.

Das Verbindungselement 34b kann weiterhin eine Positionierungseinheit 33 (nicht gezeigt) umfassen oder als ein Teil einer Positionierungseinheit 33 ausgestaltet sind. Die Positionierungseinheit 33 kann beispielsweise einen Antrieb umfassen, welcher dazu ausgebildet ist, die Gelenke 34b.1 und/oder 34.b2 anwendungsgemäß zu bewegen. Es ist vorstellbar, dass die Gelenke 34b.1 und/oder 34b.2 Getriebeelemente aufweisen, welche dazu ausgebildet sind, mittels eines in den Patientensitz 31 integrierten Motors oder eines externen Motors angetrieben zu werden. Die Positionierungseinheit 33 kann aber auch dazu ausgebildet sein, die Streben 34b.3 und 34b.4 mittels Zugstangen, Kolben oder dergleichen, relativ zueinander zu bewegen. Mittels einer oder mehrerer Positionierungseinheiten 33 kann der Patientensitz 31 teilweise oder vollständig automatisiert in eine gewünschte Konfiguration überführt werden.

Unabhängig von dem Vorhandensein einer Positionierungseinheit 33 kann der erfindungsgemäße Patientensitz 31 dazu ausgebildet sein, eine Körperregion, insbesondere den Kopf, des Patienten 15 entlang einer anterior-posterior- Richtung und einer superior-inferior-Richtung des Oberkörpers des Patienten 15 zu bewegen. Die anterior-posterior-Richtung entspricht in dem in Fig. 7 gezeigten Beispiel der Y-Richtung. Die superior-inferior-Richtung entspricht dagegen der Z-Richtung. Es ist weiterhin vorstellbar, dass die Kopfstütze 31c, aber auch die Lehne 31b, mittels eines entsprechend ausgestalteten Verbindungselements 34 und/oder einer Positionierungseinheit 33 um eine parallel zu der X-Richtung ausgerichteten Achse schwenkbar sind.

Das in Fig. 7 gezeigte Beispiel des Patientensitzes 31 weist ein Sicherungselement 61 auf, welches dazu ausgebildet ist, ein Ausmaß einer relativen Ausrichtung der Kopfstütze 31c zu der Lehne 31b zu begrenzen, um eine Kollision des Patientensitzes 31 und/oder eines anwendungsgemäß auf dem Patientensitz 31 positionierten Patienten 15 mit einem Gehäuseabschnitt eines Gehäuses 30 eines Magnetresonanzgeräts 10 zu vermeiden. Beispielsweise kann das Sicherungselement 61 dazu ausgebildet sein, ein übermäßiges Zurücklehnen des Patienten 15 auf dem Patientensitz 31 zu begrenzen, um eine Kollision des Patientensitzes 31 mit einem Gehäuse 30 wie in Fig. 8 dargestellt, zu vermeiden.

Das Sicherungselement 61 weist vorliegend eine Mehrzahl von Anschlagselementen, insbesondere Stifte oder Bolzen, auf. Die Anschlagselemente sind dazu ausgebildet, einen mittels des Gelenks 34b.1 einstellbaren Winkel α zwischen den beiden Streben 34b.4 und 34b.3 zu begrenzen. Beispielsweise können die Anschlagselemente feste Anschlagspunkte für die Streben 34b.4 und 34b.3 bilden oder einen Öffnungswinkel α des Gelenks 34b.1 begrenzen.

Selbstverständlich können auch das Gelenk 34b.2 oder ein Verbindungselement 34b.3 (nicht gezeigt), welches die Sitzfläche 31a mechanisch mit der Lehne 31b verbindet, ein Sicherungselement 61 aufweisen.

Die Fig. 8 zeigt eine Ausführungsform eines erfindungsgemäßen Magnetresonanzgeräts 10 mit einem Arretierungsmechanismus 60.

Der Arretierungsmechanismus 60 weist einen Trichter oder Konus 60a mit einer zylindrischen Vertiefung 60b und ein Koppelelement 60c mit einem kugelförmigen Ende auf. Der Konus 60a und das Koppelelement 60c sind komplementär zueinander ausgestaltet und dazu ausgebildet, mechanisch ineinanderzugreifen. Das Koppelelement 60c ist vorliegend mechanisch mit dem Patientensitz 31, insbesondere der Kopfstütze oder dem Verbindungselement 34b, verbunden. Der Konus 60a ist innerhalb des Patientenaufnahmebereichs 14 angeordnet und mechanisch mit einer Magnethaltestruktur 70 des Magnetresonanzgeräts 10 verbunden.

Der Arretierungsmechanismus 60 ist dazu ausgebildet, eine Bewegung eines Abschnitts des Patientensitzes 31 entlang einer Raumrichtung zu begrenzen. Beispielsweise verhindert der Arretierungsmechanismus 60 eine Bewegung des Patientensitzes 31 in Richtung des Endes des Patientenaufnahmebereiches 14 mit dem Arretierungsmechanismus 60, wenn das Koppelelement 60c an der Wandung in der zylindrischen Vertiefung 60b anstößt. Weiterhin kann ein Bewegungsfreiraum des kugelförmigen Endes des Koppelelements 60c entlang der Y-Richtung durch die Mantelfläche der zylindrischen Vertiefung 60b begrenzt werden. Das Koppelelement 60b kann jedoch mit einem Gelenk an einem Abschnitt des Patientensitzes 31 befestigt sein, sodass eine eingeschränkte Positionierung der Kopfstütze und/oder der Lehne des Patientensitzes entlang der Y-Richtung mittels der Verbindungselemente 34b und/oder 34a ermöglicht wird. Weiterhin kann das kugelförmige Ende des Koppelelements 60c rotierbar in der zylindrischen Vertiefung 60b gelagert sein, um eine eingeschränkte Positionierung der Kopfstütze und/oder der Lehne des Patientensitzes entlang der Y-Richtung zu ermöglichen. Die Rotation des kugelförmigen Endes des Koppelelements 60c kann dabei durch einen Öffnungswinkel des Konus 60a begrenzt sein.

In der in Fig. 8 gezeigten Ausführungsform ist der Arretierungsmechanismus 60 passiv ausgestaltet. Ein Eingreifen des Koppelelements 60c in den Konus 60a und/oder die zylindrische Vertiefung 60b wird daher im Wesentlichen durch eine Bewegung des Patientensitzes 31 entlang der Z-Richtung mittels der Antriebseinheit 31 und/oder einer Positionierungseinheit 33 realisiert.

Es ist jedoch ebenso vorstellbar, dass der Konus 60a mit der zylindrischen Vertiefung 60b und/oder das Koppelelement 60c mit einem Antrieb gekoppelt sind, welcher dazu ausgebildet ist, die beiden Teile des Arretierungsmechanismus 60 aufeinander zuzubewegen. Beispielsweise kann das Koppelelements 60c einen spindelförmig ausgestalteten Schaft aufweisen, welcher ein Positionieren des kugelförmigen Endes mittels eines Getriebes entlang der Z-Richtung ermöglicht. In einem weiteren Beispiel kann der Patientensitz 31 einen hydraulischen oder pneumatischen Antrieb, z. B. einen Kolben, aufweisen. Ein solcher Antrieb kann dazu ausgelegt sein, das Koppelelement 60c entlang der Z-Richtung auszulenken. Es ist jedoch ebenso vorstellbar, dass der Konus 60a mittels eines geeigneten Antriebs entlang der Z-Richtung und/oder der Y-Richtung positionierbar ist.

Die Figuren 4 bis 8 zeigen einen Patienten 15, welcher anwendungsgemäß auf einem erfindungsgemäßen Patientensitz 31 positioniert ist. Die hier beschriebenen Beispiele sind jedoch nicht als einschränkend hinsichtlich einer anwendungsgemäßen Position des Patienten 15 zu verstehen. Beispielsweise kann die Position und/oder die Körperhaltung des Patienten 15 in Abhängigkeit einer Konstruktion und/oder eines Designs des Patientensitzes 31 von den gezeigten Beispielen abweichen, aber dennoch als eine anwendungsgemäße Position des Patienten 15 auf dem Patientensitz 31 gelten.

Obwohl die Erfindung im Detail durch die bevorzugten Ausführungsbeispiele näher illustriert und beschrieben wurde, ist die Erfindung nicht durch die offenbarten Beispiele eingeschränkt und andere Variationen können vom Fachmann hieraus abgeleitet werden, ohne den Schutzumfang der Erfindung zu verlassen. Insbesondere können Merkmale einzelner Ausführungsformen mit Merkmalen anderer Ausführungsformen kombiniert werden, sofern eine solche Kombination nicht explizit in der Beschreibung ausgeschlossen wurde.

## Patentansprüche

1. Ein Patientensitz (31) zur Lagerung eines Patienten (15) während einer Magnetresonanzuntersuchung mittels eines Magnetresonanzgeräts (10), aufweisend einen ersten Abschnitt (31a; 31b; 31c), einen zweiten Abschnitt (31a; 31b; 31c), ein Verbindungselement (34), eine Hochfrequenzeinheit (51) mit zumindest einem Antennenelement (50), einen zweiten Teil eines Arretierungsmechanismus (60) zur Begrenzung einer Bewegung eines Abschnitts (31a; 31b; 31c) des Patientensitzes (31) entlang einer Raumrichtung, und eine Antriebseinheit (32),
• wobei der erste Abschnitt (31a; 31b; 31c) und der zweite Abschnitt (31a; 31b; 31c) Teile einer Aufnahmefläche für den Patienten (15) bilden,
• wobei das Verbindungselement (34) den ersten Abschnitt (31a; 31b; 31c) mechanisch mit dem zweiten Abschnitt (31a; 31b; 31c) verbindet und dazu ausgebildet ist, eine variable relative Bewegung zwischen dem ersten Abschnitt (31a; 31b; 31c) und dem zweiten Abschnitt (31a; 31b; 31c) zu ermöglichen,
• wobei das zumindest eine Antennenelement (50) der Hochfrequenzeinheit (51) dazu ausgebildet ist, Signale im in einem Leistungs- und Frequenzbereich einer Magnetresonanzuntersuchung zu empfangen,
• wobei der zweite Teil mechanisch mit dem Patientensitz (31) verbunden ist und komplementär zu einem innerhalb eines Patientenaufnahmebereichs (14) angeordneten und mit einem Gehäuseabschnitt des Magnetresonanzgeräts (10) verbundenen ersten Teil des Arretierungsmechanismus (60) ausgestaltet ist, wobei der zweite Teil dazu ausgebildet ist, mechanisch in den ersten Teil einzugreifen, und
• wobei die Antriebseinheit (32) dazu ausgebildet ist, den Patientensitz (31) variabel entlang einer Raumrichtung zu bewegen.

2. Der Patientensitz (31) nach Anspruch 1, aufweisend eine Positionierungseinheit (33), welche dazu ausgebildet ist, den ersten Abschnitt (31a; 31b; 31c) automatisch variabel relativ zu dem zweiten Abschnitt (31a; 31b; 31c) zu positionieren.

3. Der Patientensitz (31) nach einem der Ansprüche 1 oder 2, wobei die Antriebseinheit (32) dazu ausgebildet ist, den Patientensitz (31) variabel entlang einer ersten Raumrichtung und einer zweiten Raumrichtung, welche orthogonal zu der ersten Raumrichtung ausgerichtet ist, zu positionieren.

4. Der Patientensitz (31) nach einem der vorhergehenden Ansprüche, ferner aufweisend eine Kopfstütze (31c) und ein weiteres Verbindungselement (34), wobei das weitere Verbindungselement (34) dazu ausgebildet ist, die Kopfstütze (31c) variabel relativ zu dem ersten Abschnitt (31a; 31b) und/oder dem zweiten Abschnitt (31a; 31b) zu positionieren, und wobei die Hochfrequenzeinheit (51) an der Kopfstütze (31c) angeordnet ist.

5. Der Patientensitz (31) nach Anspruch 4, wobei das weitere Verbindungselement (34) dazu ausgebildet ist, eine variable Positionierung der Kopfstütze (31c) im Wesentlichen parallel zu einer anterior-posterior-Richtung und/oder einer superior-inferior-Richtung eines Oberkörpers eines anwendungsgemäß auf dem Patientensitz (31) positionierten Patienten (15) zu ermöglichen.

6. Der Patientensitz (31) nach einem der vorhergehenden Ansprüche, wobei das Verbindungselement (34) dazu ausgebildet ist, den ersten Abschnitt (31a; 31b; 31c) derart variabel relativ zu dem zweiten Abschnitt (31a; 31b; 31c) zu bewegen, dass eine relative Position des Kopfes eines anwendungsgemäß auf dem Patientensitz (31) positionierten Patienten (15) und eines Abschnitts (31b; 31c) des Patientensitzes (31) dabei im Wesentlichen unverändert bleibt.

7. Der Patientensitz (31) nach einem der vorhergehenden Ansprüche, wobei die Hochfrequenzeinheit (51) ein Führungselement (53) aufweist, und wobei das Führungselement (53) dazu ausgebildet ist, das zumindest eine Antennenelement (50) variabel relativ zu einem Abschnitt (31a; 31b; 31c) des Patientensitzes (31) zu bewegen.

8. Der Patientensitz (31) nach einem der vorhergehenden Ansprüche, wobei die Hochfrequenzeinheit (51) einen Schwenkmechanismus (52) aufweist, welcher dazu ausgebildet ist, das zumindest eine Antennenelement (50) um eine bei anwendungsgemäßer Positionierung des Patienten (15) auf dem Patientensitz (31) im Wesentlichen parallel zu einer medialen Richtung des Patienten (15) ausgerichteten Achse zu schwenken.

9. Ein Magnetresonanzgerät (10) zum Durchführen einer Magnetresonanzuntersuchung eines in einem Patientenaufnahmebereich (14) des Magnetresonanzgeräts (10) angeordneten Patienten (15), aufweisend einen Patientensitz (31) nach einem der vorhergehenden Ansprüche sowie einen Arretierungsmechanismus (60) mit einem ersten Teil und einem zweiten Teil, wobei der erste Teil und der zweite Teil komplementär zueinander ausgestaltet sind und dazu ausgebildet sind, mechanisch ineinanderzugreifen, wobei der zweite Teil mechanisch mit dem Patientensitz (31) verbunden ist und wobei der erste Teil innerhalb des Patientenaufnahmebereichs (14) angeordnet und mit einem Gehäuseabschnitt des Magnetresonanzgeräts (10) mechanisch verbunden ist, wobei der Arretierungsmechanismus (60) dazu ausgebildet ist, eine Bewegung eines Abschnitts (31a; 31b; 31c) des Patientensitzes (31) entlang einer Raumrichtung zu begrenzen, wobei die Antriebseinheit (32) dazu ausgebildet ist, den Patientensitz (31) zumindest entlang einer Raumrichtung variabel relativ zu dem Patientenaufnahmebereich (14) des Magnetresonanzgeräts (10) zu bewegen.

10. Das Magnetresonanzgerät (10) nach Anspruch 9, aufweisend ein Sicherungselement (61), welches dazu ausgebildet ist, ein Ausmaß einer relativen Ausrichtung des ersten Abschnitts (31a; 31b; 31c) zu dem zweiten Abschnitt (31a; 31b; 31c) zu begrenzen, um eine Kollision des Patientensitzes (31) und/oder eines anwendungsgemäß auf dem Patientensitz (31) positionierten Patienten (15) mit einem Gehäuseabschnitt des Magnetresonanzgeräts (10) zu vermeiden.

11. Das Magnetresonanzgerät (10) nach einem der Ansprüche 9 oder 10 mit einem Patientensitz (31) nach Anspruch 2, wobei die Antriebseinheit (32) und die Positionierungseinheit (33) dazu ausgebildet sind, den Patientensitz (31) entlang der Raumrichtung dem Patientenaufnahmebereich (14) des Magnetresonanzgeräts (10) zuzuführen und gleichzeitig den ersten Abschnitt (31a; 31b; 31c) variabel relativ zu dem zweiten Abschnitt (31a; 31b; 31c) zu bewegen.

12. Das Magnetresonanzgerät (10) nach einem der Ansprüche 9 bis 11 mit einem Patientensitz (31) nach Anspruch 2, wobei die Antriebseinheit (32) und/oder die Positionierungseinheit (33) dazu ausgebildet sind, eine diagnostische relevante Körperregion eines anwendungsgemäß auf dem Patientensitz (31) positionierten Patienten (15) zumindest entlang einer ersten Raumrichtung und einer zweiten Raumrichtung, welche orthogonal zu der ersten Raumrichtung ausgerichtet ist, automatisch in einem Bildgebungsvolumen (35) des Magnetresonanzgeräts (10) zu positionieren.

13. Das Magnetresonanzgerät (10) nach Anspruch 12, aufweisend einen Sensor (40), welcher dazu ausgebildet ist, eine Information über eine relative räumliche Anordnung des zumindest einen Antennenelements zu einem Abschnitt (31a; 31b; 31c) des Patientensitzes (31) zu bestimmen, wobei die Antriebseinheit (32) und/oder die Positionierungseinheit (33) des Patientensitzes (31) dazu ausgebildet sind, die diagnostisch relevante Körperregion des Patienten (15) in Abhängigkeit der Information über die relative räumliche Anordnung des zumindest einen Antennenelements (50) zu dem Abschnitt (31a; 31b; 31c) in dem Bildgebungsvolumen (35) zu positionieren.

14. Das Magnetresonanzgerät (10) nach einem der Ansprüche 9 bis 13, wobei der Arretierungsmechanismus (60) einen Antrieb aufweist, welcher dazu ausgebildet ist, in Abhängigkeit eines Aktivierungssignals ein Ineinandergreifen des ersten Teils und des zweiten Teils zu bewirken, wenn sich der Patientensitz (31) in einer anwendungsgemäßen Position relativ zu dem Patientenaufnahmebereich (14) zur Durchführung der Magnetresonanzuntersuchung befindet.

## Claims

1. Patient seat (31) for supporting a patient (15) during a magnetic resonance examination by means of a magnetic resonance device (10), having a first portion (31a; 31b; 31c), a second portion (31a; 31b; 31c), a connecting element (34), a radio-frequency unit (51) with at least one antenna element (50), a second part of a locking mechanism (60) for limiting a movement of a portion (31a; 31b; 31c) of the patient seat (31) along a spatial direction, and a drive unit (32),
• wherein the first portion (31a; 31b; 31c) and the second portion (31a; 31b; 31c) form parts of a receiving surface for the patient (15),
• wherein the connecting element (34) mechanically connects the first portion (31a; 31b; 31c) to the second portion (31a; 31b; 31c) and is embodied to enable a variable relative movement between the first portion (31a; 31b; 31c) and the second portion (31a; 31b; 31c),
• wherein the at least one antenna element (50) of the radio-frequency unit (51) is embodied to receive signals in a power and frequency range of a magnetic resonance examination,
• wherein the second part is mechanically connected to the patient seat (31) and is designed as complementary to a first part of the locking mechanism (60) arranged within a patient receiving region (14) and connected to a housing portion of the magnetic resonance device (10), wherein the second part is embodied to engage mechanically with the first part, and
• wherein the drive unit (32) is embodied to move the patient seat (31) variably along a spatial direction.

2. Patient seat (31) according to claim 1, having a positioning unit (33), which is embodied to automatically position the first portion (31a; 31b; 31c) variably relative to the second portion (31a; 31b; 31c).

3. Patient seat (31) according to one of claims 1 or 2, wherein the drive unit (32) is embodied to position the patient seat (31) variably along a first spatial direction and a second spatial direction, which is aligned orthogonally to the first spatial direction.

4. Patient seat (31) according to one of the preceding claims, furthermore having a headrest (31c) and a further connecting element (34), wherein the further connecting element (34) is embodied to position the headrest (31c) variably relative to the first portion (31a; 31b) and/or the second portion (31a; 31b), and wherein the radio-frequency unit (51) is arranged on the headrest (31c).

5. Patient seat (31) according to claim 4, wherein the further connecting element (34) is embodied to enable variable positioning of the headrest (31c) substantially parallel to an anterior-posterior direction and/or a superior-inferior direction of an upper body of a patient (15) positioned on the patient seat (31) appropriately for the application.

6. Patient seat (31) according to one of the preceding claims, wherein the connecting element (34) is embodied to move the first portion (31a; 31b; 31c) variably relative to the second portion (31a; 31b; 31c) such that a relative position of the head of a patient (15) positioned on the patient seat (31) appropriately for the application and a portion (31b; 31c) of the patient seat (31) remains substantially unchanged.

7. Patient seat (31) according to one of the preceding claims, wherein the radio-frequency unit (51) has a guide element (53), and wherein the guide element (53) is embodied to move the at least one antenna element (50) variably relative to a portion (31a; 31b; 31c) of the patient seat (31).

8. Patient seat (31) according to one of the preceding claims, wherein the radio-frequency unit (51) has a pivoting mechanism (52), which is embodied to pivot the at least one antenna element (50) about an axis aligned substantially parallel to a medial direction of the patient (15) when the patient (15) is positioned on the patient seat (31) appropriately for the application.

9. Magnetic resonance device (10) for performing a magnetic resonance examination on a patient (15) arranged in a patient receiving region (14) of the magnetic resonance device (10), having a patient seat (31) according to one of the preceding claims, and a locking mechanism (60) with a first part and a second part, wherein the first part and the second part are designed as complementary to one another and are embodied to mechanically engage with one another, wherein the second part is mechanically connected to the patient seat (31) and wherein the first part is arranged within the patient receiving region (14) and mechanically connected to a housing portion of the magnetic resonance device (10), wherein the locking mechanism (60) is embodied to limit a movement of a portion (31a; 31b; 31c) of the patient seat (31) along a spatial direction, wherein the drive unit (32) is embodied to move the patient seat (31) variably relative to the patient receiving region (14) of the magnetic resonance device (10) at least along a spatial direction.

10. Magnetic resonance device (10) according to claim 9, having a securing element (61), which is embodied to limit an extent of an alignment of the first portion (31a; 31b; 31c) relative to the second portion (31a; 31b; 31c) in order to avoid a collision of the patient seat (31) and/or a patient (15) positioned on the patient seat (31) appropriately for the application with a housing portion of the magnetic resonance device (10).

11. Magnetic resonance device (10) according to one of claims 9 or 10 with a patient seat (31) according to claim 2, wherein the drive unit (32) and the positioning unit (33) are embodied to feed the patient seat (31) along the spatial direction to the patient receiving region (14) of the magnetic resonance device (10) and to simultaneously move the first portion (31a; 31b; 31c) variably relative to the second portion (31a; 31b; 31c).

12. Magnetic resonance device (10) according to one of claims 9 to 11 with a patient seat (31) according to claim 2, wherein the drive unit (32) and/or the positioning unit (33) are embodied to automatically position a diagnostically relevant body region of a patient (15) positioned on the patient seat (31) appropriately for the application at least along a first spatial direction and a second spatial direction, which is aligned orthogonally to the first spatial direction in an imaging volume (35) of the magnetic resonance device (10).

13. Magnetic resonance device (10) according to claim 12, having a sensor (40), which is embodied to determine information about a spatial arrangement of the at least one antenna element relative to a portion (31a; 31b; 31c) of the patient seat (31), wherein the drive unit (32) and/or the positioning unit (33) of the patient seat (31) are embodied to position the diagnostically relevant body region of the patient (15) in the imaging volume (35) in dependence on the information about the spatial arrangement of the at least one antenna element (50) relative to the portion (31a; 31b; 31c).

14. Magnetic resonance device (10) according to one of claims 9 to 13, wherein the locking mechanism (60) has a drive, which is embodied to effect an engagement of the first part and the second part in dependence on an activation signal when the patient seat (31) is located in an application-appropriate position relative to the patient receiving region (14) for performing the magnetic resonance examination.

## Revendications

1. Un siège (31) de patient pour l'installation d'un patient (15) pendant un examen par résonnance magnétique au moyen d'un appareil (10) par résonnance magnétique, comportant une première partie (31a ; 31b ; 31c), une deuxième partie (31a ; 31b ; 31c), un élément (34) de liaison, une unité (51) à haute fréquence ayant au moins un élément (50) d'antenne, une deuxième partie d'un mécanisme (60) d'arrêt pour la limitation d'un mouvement d'une partie (31a, ; 31b ; 31c) du siège (31) du patient suivant une direction de l'espace et une unité (32) d'entraînement,
• dans lequel la première partie (31a ; 31b ; 31c) et la deuxième partie (31a ; 31b ; 31c) forment des parties d'une surface d'enregistrement pour le patient (15),
• dans lequel l'élément (34) de liaison relie la première partie (31a ; 31b ; 31c) mécaniquement à la deuxième partie (31a ; 31b ; 31c) et est constitué pour permettre un mouvement relatif variable entre la première partie (31a ; 31b ; 31c) et la deuxième partie (31a ; 31b ; 31c),
• dans lequel le au moins un élément (50) d'antenne de l'unité (51) à haute fréquence est constitué pour recevoir des signaux d'un domaine de puissance et de fréquence d'un examen par résonnance magnétique,
• dans lequel la deuxième partie est reliée mécaniquement au siège (31) de patient et est conformée de manière complémentaire à une première partie du mécanisme (60) d'arrêt disposée à l'intérieur d'une zone (14) d'enregistrement du patient et reliée à une partie d'enveloppe de l'appareil (10) par résonnance magnétique, dans lequel la deuxième partie est constituée pour pénétrer mécaniquement dans la première partie, et
• dans lequel l'unité (32) d'entraînement est constituée pour déplacer le siège (31) de patient de manière variable suivant une direction dans l'espace.

2. Le siège (31) de patient de la revendication 1, comportant une unité (33) de mise en position, qui est constituée pour mettre en position la première partie (31a ; 31b ; 31c) automatiquement de manière variable par rapport à la deuxième partie (31a ; 31b ; 31c).

3. Le siège (31) de patient de l'une des revendication 1 ou 2, dans lequel l'unité (32) d'entraînement est constituée pour mettre en position le siège (31) de patient de manière variable suivant une première direction dans l'espace et une deuxième direction dans l'espace, qui est orthogonale à la première direction dans l'espace.

4. Le siège (31) de patient de l'une des revendications précédentes, comportant en outre un appui-tête (31c) et un autre élément (34) de liaison, dans lequel l'autre élément (34) de liaison est constitué pour mettre en position l'appui-tête (31c) de manière variable par rapport à la première partie (31a ; 31b) et/ou la deuxième partie (31a ; 31b), et dans lequel l'unité (51) à haute fréquence est montée sur l'appui-tête (31c).

5. Le siège (31) de patient de la revendication 4, dans lequel l'autre élément (34) de liaison est constitué pour permettre une mise en position variable de l'appui-tête (31c) sensiblement parallèlement à une direction antérieure - postérieure et/ou à une direction supérieure - inférieure d'un buste d'un patient (15) mis en position, suivant l'application, sur le siège (31) de patient.

6. Le siège (31) de patient de l'une des revendications précédentes, dans lequel l'élément (34) de liaison est constitué pour déplacer la première partie (31a ; 31b ; 31c), de manière variable par rapport à la deuxième partie (31a ; 31b ; 31c), de manière à ce qu'une position relative de la tête d'un patient (15) mise en position, conformément à l'application, sur le siège (31) de patient et une partie (31b ; 31c) du siège (31) du patient reste sensiblement inchangée.

7. Le siège (31) de patient de l'une des revendications précédentes, dans lequel l'unité (51) à haute fréquence a un élément (53) de guidage, et dans lequel l'élément (53) de guidage est constitué pour déplacer la au moins une unité (50) d'antenne de manière variable par rapport à une partie (31a ; 31b ; 31c) du siège (31) du patient.

8. Le siège (31) de patient de l'une des revendications précédentes, dans lequel l'unité (51) à haute fréquence a un mécanisme (52) de pivotement, qui est constitué pour faire pivoter le au moins un élément (50) d'antenne autour d'un axe dirigé lors de la mise en position du patient (15) sur le siège (31) de patient, conforme à l'application, sensiblement parallèlement à une direction médiane du patient (15).

9. Un appareil (10) par résonnance magnétique pour effectuer un examen par résonnance magnétique d'un patient (15) disposé dans une partie (14) d'enregistrement du patient de l'appareil (10) par résonnance magnétique, comportant un siège (31) de patient suivant l'une des revendications précédentes, ainsi qu'un mécanisme (60) d'arrêt comprenant une première partie et une deuxième partie, dans lequel la première partie et la deuxième partie sont conformées de manière complémentaire l'une de l'autre et sont constituées pour se prendre entre elles mécaniquement, dans lequel la deuxième partie est reliée mécaniquement au siège (31) de patient et dans lequel la première partie est disposée à l'intérieur de la zone (14) d'enregistrement de patient et est reliée mécaniquement à une partie de boîtier de l'appareil (10) par résonnance magnétique, dans lequel le mécanisme (60) d'arrêt est constitué pour limiter un mouvement d'une partie (31a ; 31b ; 31c) du siège (31) de patient suivant une direction de l'espace, dans lequel l'unité (32) d'entraînement est constituée pour déplacer le siège (31) de patient au moins suivant une direction de l'espace de manière variable par rapport à la zone (14) d'enregistrement de patient de l'appareil (10) par résonnance magnétique.

10. L'appareil (10) par résonnance magnétique de la revendication 9, comportant un élément (61) de sécurisation, qui est constitué pour limiter l'étendue d'une orientation relative de la première partie (31a ; 31b ; 31c) par rapport à la deuxième partie (31a ; 31b ; 31c), afin d'empêcher une collision du siège (31) de patient et/ou d'un patient (15) mis en position, d'une manière conforme à l'application, sur le siège (31) de patient avec une partie du boîtier de l'appareil (10) par résonnance magnétique.

11. L'appareil (10) par résonnance magnétique de l'une des revendications 9 ou 10 comprenant un siège (31) de patient suivant la revendication 2, dans lequel l'unité (32) d'entraînement et l'unité (33) de mise en position sont constituées pour apporter le siège (31) de patient suivant la direction de l'espace à la zone (14) d'enregistrement de patient de l'appareil (10) par résonnance magnétique, et pour déplacer, en même temps, la première partie (31a ; 31b ; 31c) de manière variable par rapport à la deuxième partie (31a ; 31b ; 31c).

12. L'appareil (10) par résonnance magnétique de l'une des revendications 9 à 11 comprenant un siège (31) de patient suivant la revendication 2, dans lequel l'unité (32) d'entraînement et/ou l'unité (33) de mise en position sont constituées pour mettre en position automatiquement, dans un volume (35) d'imagerie de l'appareil (10) par résonnance magnétique, une région du corps pertinente pour le diagnostic d'un patient (15) mis en position, conformément à l'application, sur le siège (31) de patient, au moins suivant une première direction dans l'espace et une deuxième direction dans l'espace, qui est orthogonale à la première direction dans l'espace.

13. L'appareil (10) par résonnance magnétique de la revendication 12, comportant un capteur (40), qui est constitué pour déterminer une information sur une disposition relative dans l'espace du au moins un élément d'antenne par rapport à une partie (31a ; 31b ; 31c) du siège (31) de patient, dans lequel l'unité (32) d'entraînement et/ou l'unité (33) de mise en position du siège (31) du patient sont constituées pour mettre en position, dans le volume (35) d'imagerie, la région du corps pertinente pour le diagnostic du patient (15) en fonction de l'information sur l'agencement relatif dans l'espace du au moins un élément (50) d'antenne par rapport à la partie (31a ; 31b ; 31c).

14. L'appareil (10) par résonnance magnétique de l'une des revendications 9 à 13, dans lequel le mécanisme (60) d'arrêt a un entraînement, qui est constitué pour provoquer, en fonction d'un signal d'activation, une prise entre la première partie et la deuxième partie, lorsque le siège (31) de patient se trouve dans une position, conforme à l'application, par rapport à la zone (14) d'enregistrement de patient pour effectuer l'examen par résonnance magnétique.
